# DEMANDE DE BREVET EUROPEEN

(11) **EP 1 020 176 A2**
(43) Date de publication de la demande: **19.07.2000**
(21) Numéro de dépôt: 99403167.2
(22) Date de dépôt: 16.12.1999
(51) Int. Cl.: A61K 7/13

(54) **Utilisation de composés nitres di-benzéniques cationiques en teinture des fibres kératiniques, compositions tinctoriales et procédés de teinture**

(30) Priorité: 08.01.1999 FR 9900149
(71) Demandeur: L'OREAL, 75008 Paris (FR)
(72) Inventeur: Genet, Alain, 93600 Aulnay Sous Bois (FR); Lagrange, Alain, 77700 Coupvray (FR)
(74) Mandataire: Miszputen, Laurent

(57) **Abrégé**

L'invention a pour objet l'utilisation de composés nitrés di-benzéniques comportant au moins un groupement cationique Z, Z étant choisi parmi des chaînes aliphatiques quaternisées, des chaînes aliphatiques comportant au moins un cycle saturé quaternisé et des chaînes aliphatiques comportant au moins un cycle insaturé quaternisé, à titre de colorants directs dans des compositions destinées à la teinture des matières kératiniques et en particulier des compositions destinées à la teinture des fibres kératiniques humaines et notamment des cheveux, les compositions de teinture les contenant et le procédé de teinture directe les mettant en oeuvre.

## Description

L'invention a pour objet l'utilisation de composés nitrés di-benzéniques comportant au moins un groupement cationique Z, Z étant choisi parmi des chaînes aliphatiques quaternisées, des chaînes aliphatiques comportant au moins un cycle saturé quaternisé et des chaînes aliphatiques comportant au moins un cycle insaturé quaternisé, à titre de colorants directs dans des compositions destinées à la teinture des matières kératiniques et en particulier des compositions destinées à la teinture des fibres kératiniques humaines et notamment des cheveux, les compositions de teinture les contenant et le procédé de teinture directe correspondant.

Dans le domaine de la teinture capillaire, on recherche des colorants directs, c'est-à-dire des colorants qui, sans l'apport d'agent oxydant autre que l'air ambiant, sont capables de modifier par eux-mêmes la nuance naturelle des cheveux, de façon temporaire. Dans cette application, les colorants doivent satisfaire à un certain nombre de critères, et notamment engendrer des teintures reproductibles avec des nuances riches et variées permettant d'obtenir une large palette de couleurs susceptible de satisfaire le formulateur, ces teintures devant en outre être puissantes et résistantes au lavage, au frottement, à la lumière et à la transpiration.

Or, la demanderesse vient maintenant de découvrir, de façon totalement inattendue et surprenante, que des composés nitrés di-benzéniques de formule (I) ci-après définie, comportant au moins un groupement cationique Z, Z étant choisi parmi des chaînes aliphatiques quaternisées, des chaînes aliphatiques comportant au moins un cycle saturé quaternisé, et des chaînes aliphatiques comportant au moins un cycle insaturé quaternisé, conviennent pour une utilisation comme colorant direct pour la teinture directe, mais en outre qu'ils permettent d'obtenir des compositions tinctoriales conduisant à des colorations puissantes et variées présentant d'excellentes propriétés de résistance aux différents traitements que peuvent subir les fibres kératiniques. Enfin, ces composés présentent une meilleure solubilité dans les milieux classiquement utilisés pour la teinture des fibres kératiniques et s'avèrent être aisément synthétisables.

Ces découvertes sont à la base de la présente invention.

L'invention a donc pour premier objet, l'utilisation, à titre de colorants directs dans des, ou pour la fabrication de, compositions tinctoriales pour matières kératiniques, et notamment pour fibres kératiniques humaines telles que les cheveux , de composés nitrés di-benzéniques de formule (I) suivante, et leurs sels d'addition avec un acide : 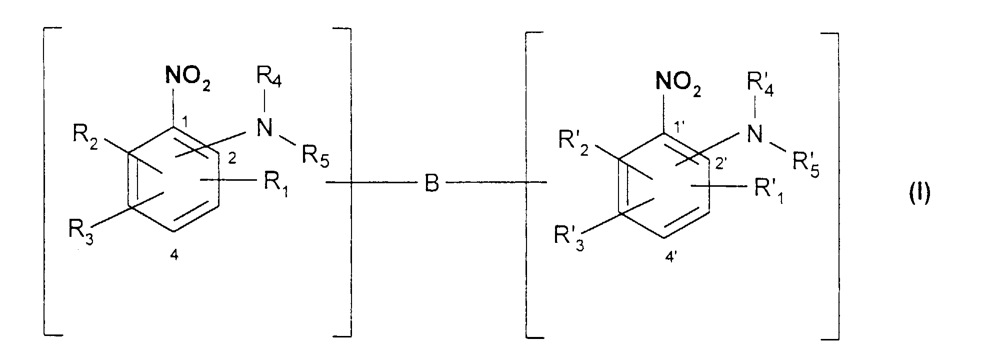 dans laquelle :
- B est un bras de liaison qui représente une chaîne alkyle comportant de préférence de 1 à 14 atomes de carbone, linéaire ou ramifiée pouvant être interrompue par un ou plusieurs groupements Z et/ou par un ou plusieurs hétéroatomes tels que des atomes d'oxygène, de soufre ou d'azote, et éventuellement substituée par un ou plusieurs radicaux hydroxyle ou alcoxy en C₁-C₆, et pouvant porter une ou plusieurs fonctions cétone ;
- R₁, R₂, R₃, R'₁, R'₂ et R'₃, qui peuvent être identiques ou différents, représentent l'une des deux valences d'un bras de liaison B, un atome d'hydrogène ; un atome d'halogène ; un groupement Z ; un radical alkyl(C₁-C₆) carbonyle ; un radical aminoalkyl(C₁-C₆)carbonyle ; un radical N-Z-aminoalkyl(C₁-C₆)carbonyle ; un radical N-alkyl(C₁-C₆)aminoalkyl(C₁-C₆)carbonyle; un radical N,N-dialkyl(C₁-C₆)aminoalkyl(C₁-C₆)carbonyle ; un radical aminoalkyl(C₁-C₆)carbonylalkyle(C₁-C₆) ; un radical N-Z-aminoalkyl(C₁-C₆)carbonylalkyle(C₁-C₆) ; un radical N-alkyl(C₁-C₆)aminoalkyl(C₁-C₆)carbonylalkyle(C₁-C₆) ; un radical N,N-dialkyl(C₁-C₆)aminoalkyl(C₁-C₆)carbonylalkyle(C₁-C₆) ; un radical carboxy ; un radical alkyl(C₁-C₆) carboxy ; un radical alkyl(C₁-C₆) sulfonyle ; un radical aminosulfonyle ; un radical N-Z-aminosulfonyle ; un radical N-alkyl(C₁-C₆)aminosulfonyle ; un radical N,N-dialkyl(C₁-C₆)aminosulfonyle ; un radical aminosulfonylalkyle(C₁-C₆); un radical N-Z-aminosulfonylalkyle(C₁-C₆); un radical N-alkyl(C₁-C₆)aminosulfonylalkyle(C₁-C₆) ; un radical N,N-dialkyl(C₁-C₆)aminosulfonylalkyle(C₁-C₆) ; un radical carbamyle ; un radical N-alkyl(C₁-C₆)carbamyle ; un radical N,N-dialkyl(C₁-C₆)carbamyle ; un radical carbamylalkyle(C₁-C₆); un radical N-alkyl(C₁-C₆)carbamylalkyle(C₁-C₆) ; un radical N,N-dialkyl(C₁-C₆)carbamylalkyle(C₁-C₆) ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical trifluoroalkyle en C₁-C₆ ; un radical cyano ; un radical amino substitué ou non substitué, avec les mêmes significations que dans le groupement -NR₄R₅ ou -NR'₄R'₅ décrits ci-dessous, et pouvant être identique ou différent ; un radical aminoalkyle(C₁-C₆) dont l'alkyle est substitué ou non substitué par un ou plusieurs radicaux hydroxy et dont l'amine est substituée par un ou deux radicaux, identiques ou différents, choisis parmi les radicaux alkyle en C₁-C₆, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle, ou par un groupement Z ; un groupement OR₆ ou -SR₆
- R₅ désigne l'une des deux valences d'un bras de liaison B, un atome d'hydrogène ; un radical alkyle en C₁-C₆; un radical monohydroxyalkyle en C₁-C₆ ; un radical polyhydroxyalkyle en C₂-C₆ ; un groupement Z ; un radical alcoxy(C₁-C₆)alkyle en C₁-C₆ ; un radical aryle ; un radical benzyle ; un radical carboxyalkyle en C₁-C₆ ; un radical alkyl(C₁-C₆)carboxyalkyle en C₁-C₆ ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical trifluoroalkyle en C₁-C₆ ; un radical aminosulfonylalkyle en C₁-C₆ ; un radical N-Z-aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical aminoalkyle en (C₁-C₆) ; un radical aminoalkyle en (C₁-C₆) dont l'amine est substituée par un ou deux radicaux, identiques ou différents, choisis parmi les radicaux alkyle(C₁-C₆), monohydroxyalkyle(C₁-C₆), polyhydroxyalkyle(C₂-C₆), alkyl(C₁-C₆)carbonyle, formyle, trifluoroalkyl(C₁-C₆)carbonyle, alkyl(C₁-C₆)carboxy, carbamyle, N-alkyl(C₁-C₆)carbamyle, N,N-dialkyl(C₁-C₆)carbamyle, thiocarbamyle, alkyl(C₁-C₆)sulfonyle, ou par un groupement Z ;
- R₄, R₅, R'₄, R'₅, identiques ou différents, représentent l'une des deux valences d'un bras de liaison B, un atome d'hydrogène ; un groupement Z ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical thiocarbamylalkyle en C₁-C₆ ; un radical trifluoroalkyle en C₁-C₆ ; un radical sulfoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical aminosulfonylalkyle en C₁-C₆ ; un radical N-Z-aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)carbonylalkyle en C₁-C₆ ; un radical aminoalkyle en C₁-C₆ ; un radical aminoalkyle(C₁-C₆) dont l'alkyle est substitué ou non substitué par un ou plusieurs radicaux hydroxy et dont l'amine est substituée par un ou deux radicaux, identiques ou différents, choisis parmi les radicaux alkyle, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle, ou par un groupement Z ;
- Z est choisi parmi les groupements cationiques insaturés de formules (II) et (III) suivantes, et les groupements cationiques saturés de formule (IV) suivante 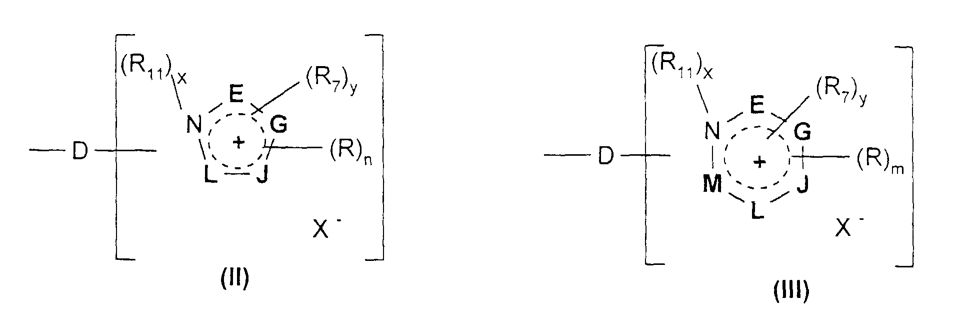 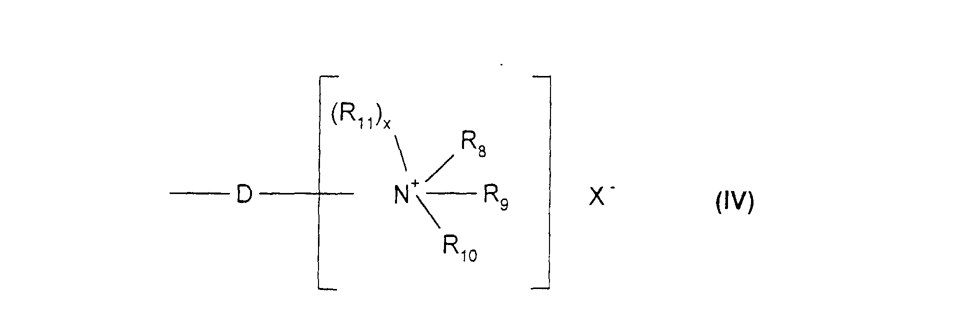
dans lesquelles :
- D est un bras de liaison qui représente une chaîne alkyle comportant de préférence de 1 à 14 atomes de carbone, linéaire ou ramifiée pouvant être interrompue par un ou plusieurs hétéroatomes tels que des atomes d'oxygène, de soufre ou d'azote, et pouvant être substituée par un ou plusieurs radicaux hydroxyle ou alcoxy en C₁-C₆, et pouvant porter une ou plusieurs fonctions cétone ;
- les sommets E, G, J, L et M, identiques ou différents, représentent un atome de carbone, d'oxygène, de soufre ou d'azote ;
- n est un nombre entier compris entre 0 et 4 inclusivement ;
- m est un nombre entier compris entre 0 et 5 inclusivement ;
- les radicaux R, identiques ou différents, représentent l'une des deux valences d'un bras de liaison B, un second groupement Z identique ou différent du premier groupement Z, un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhyde, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle; un groupement NHR" ou NR"R"' dans lesquels R" et R"', identiques ou différents, représentent un radical alkyle en C₁-C₆, un radical monohydroxyalkyle en C₁-C₆ ou un radical polyhydroxyalkyle en C₂-C₆ ;
- R₇ représente l'une des deux valences d'un bras de liaison B, un radical alkyle en C₁-C₆, un radical monohydroxyalkyle en C₁-C₆, un radical polyhydroxyalkyle en C₂-C_{6,} un radical cyanoalkyle en C₁-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle, un second groupement Z identique ou différent du premier groupement Z ;
- R₈, R₉ et R₁₀, identiques ou différents, représentent l'une des deux valences d'un bras de liaison B, un radical alkyle en C₁-C₆, un radical monohydroxyalkyle en C₁-C₆, un radical polyhydroxyalkyle en C₂-C₆, un radical alcoxy(C₁-C₆)alkyle en C₁-C₆, un radical cyanoalkyle en C₁-C₆, un radical aryle, un radical benzyle, un radical amidoalkyle en C₁-C₆, un radical trialkyl(C₁-C₆)silanealkyle en C₁-C₆ ou un radical aminoalkyle en C₁-C₆ dont l'amine est protégée par un radical alkyl(C₁-C₆)carbonyle, carbamyle, ou alkyl(C₁-C₆)sulfonyle ; deux des radicaux R₈, R₉ et R₁₀ peuvent également former ensemble, avec l'atome d'azote auquel ils sont rattachés, un cycle saturé à 5 ou 6 chaînons carboné pouvant contenir un ou plusieurs hétéroatomes tel que par exemple un cycle pyrrolidine, un cycle pipéridine, un cycle pipérazine ou un cycle morpholine, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical cétoalkyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ;
   l'un des radicaux R₈, R₉ et R₁₀ peut également représenter un second groupement Z, identique ou différent du premier groupement Z ;
- R₁₁ représente l'une des deux valences d'un bras de liaison B, un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est protégée par un radical alkyl(C₁-C₆)carbonyle, carbamyle ou alkyl(C₁-C₆)sulfonyle ; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
- x et y sont des nombres entiers égaux à 0 ou 1 ; avec les conditions suivantes :
   - dans les groupements cationiques insaturés de formule (II) :
      - lorsque x = 0, le bras de liaison D est rattaché à l'atome d'azote,
      - lorsque x = 1, le bras de liaison D est rattaché à l'un des sommets E, G, J ou L,
      - y ne peut prendre la valeur 1 que :
         1) lorsque les sommets E, G, J et L représentent simultanément un atome de carbone, et que le radical R₇ est porté par l'atome d'azote du cycle insaturé ; ou bien
         2) lorsqu'au moins un des sommets E, G, J et L représente un atome d'azote sur lequel le radical R₇ est fixé ;
   - dans les groupements cationiques insaturés de formule (III):
      - lorsque x = 0, le bras de liaison D est rattaché à l'atome d'azote,
      - lorsque x = 1, le bras de liaison D est rattaché à l'un des sommets E, G, J, L ou M,
      - y ne peut prendre la valeur 1 que lorsqu'au moins un des sommets E, G, J, L et M représente un atome divalent, et que le radical R₇ est porté par l'atome d'azote du cycle insaturé ;
   - dans les groupements cationiques de formule (IV) :
      - lorsque x = 0, alors le bras de liaison D est rattaché à l'atome d'azote portant les radicaux R₈ à R₁₀,
      - lorsque x = 1, alors deux des radicaux R₈ à R₁₀ forment conjointement avec l'atome d'azote auquel ils sont rattachés un cycle saturé à 5 ou 6 chaînons tel que défini précédemment; et le bras de liaison D est porté par un atome de carbone dudit cycle saturé ;
- X⁻ représente un anion monovalent ou divalent et est de préférence choisi parmi un atome d'halogène tel que le chlore, le brome, le fluor ou l'iode, un hydroxyde, un hydrogènesulfate, ou un alkyl(C₁-C₆)sulfate tel que par exemple un méthylsulfate ou un éthylsulfate ;
**étant entendu que** le nombre de groupement cationique Z est au moins égal à 1.

Dans les formules (I), (II), (III) et (IV) ci-dessus les radicaux alkyle et alcoxy peuvent être linéaires ou ramifiés.
Les composés de formule (I) peuvent être éventuellement salifiés par des acides minéraux forts tels que HCI, HBr, H₂SO₄, ou des acides organiques tels qu'acétique, tartrique, lactique, citrique ou succinique.

Parmi les cycles des groupements insaturés Z de formule (Il) ci-dessus, on peut notamment citer à titre d'exemple les cycles pyrrolique, imidazolique, pyrazolique, oxazolique, thiazolique et triazolique.

Parmi les cycles des groupements insaturés Z de formule (III) ci-dessus, on peut notamment citer à titre d'exemple les cycles pyridinique, pyrimidinique, pyrazinique, oxazinique et triazinique.

Parmi les composés de formule (I) ci-dessus, on peut notamment citer les composés de formules (I)₁ à (I)₂₃ suivantes :

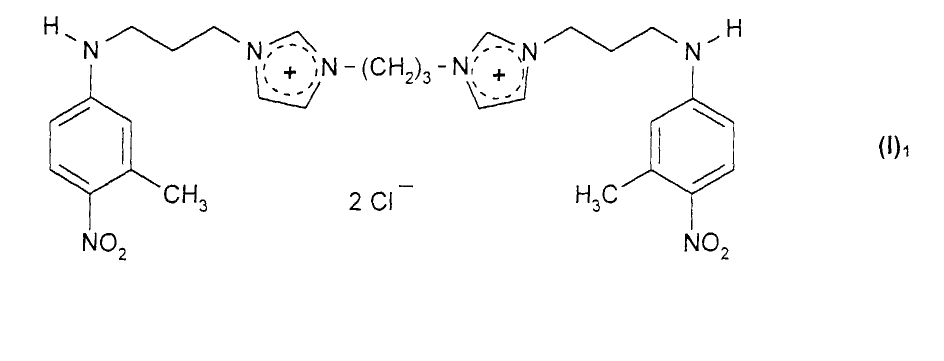

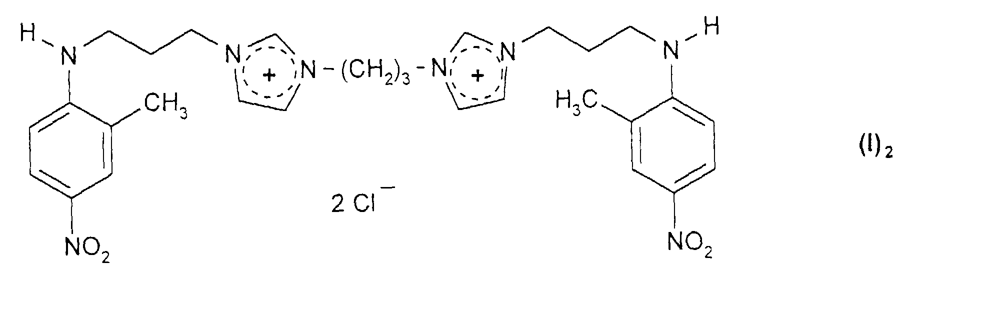

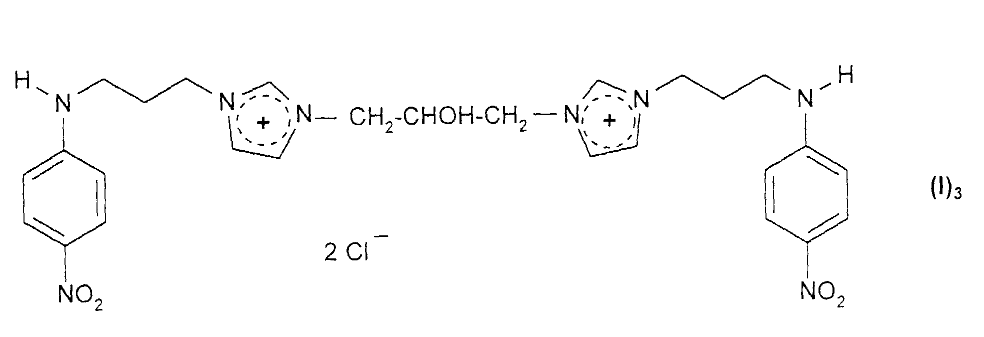

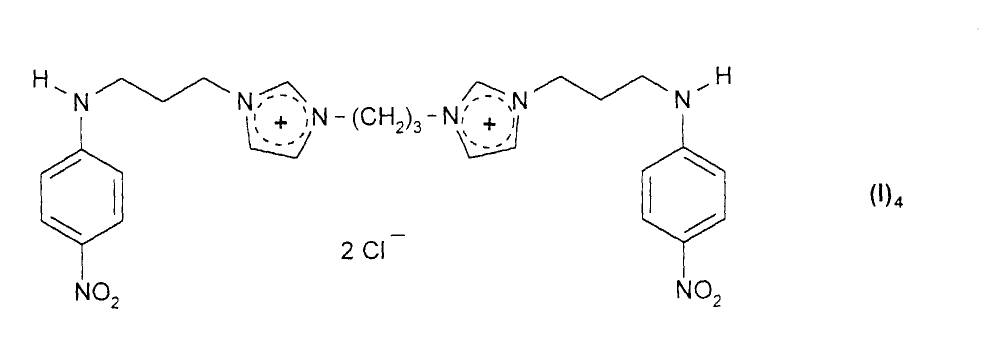

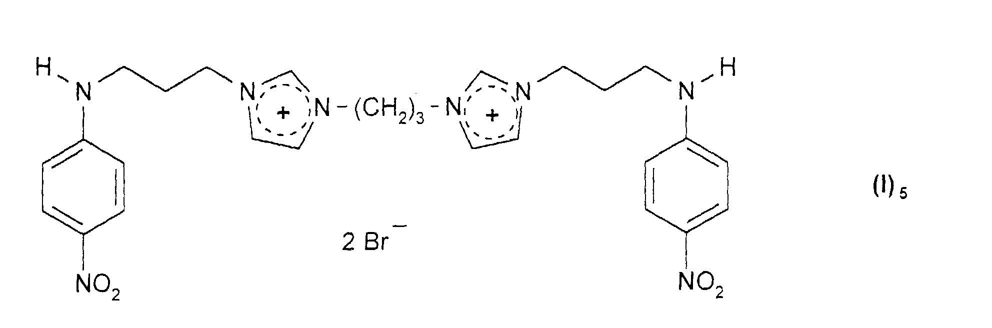

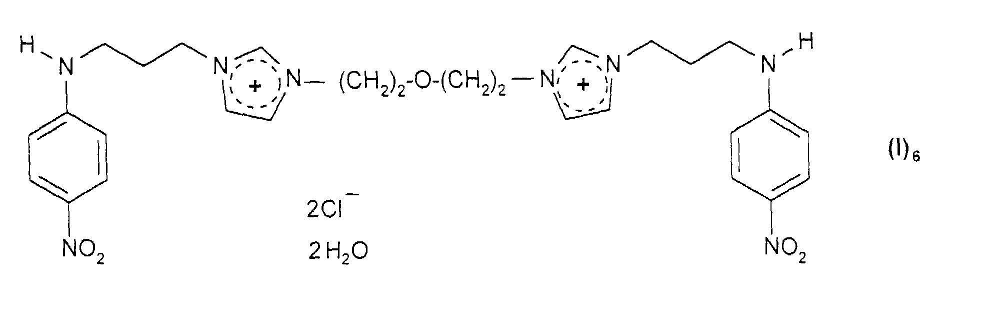

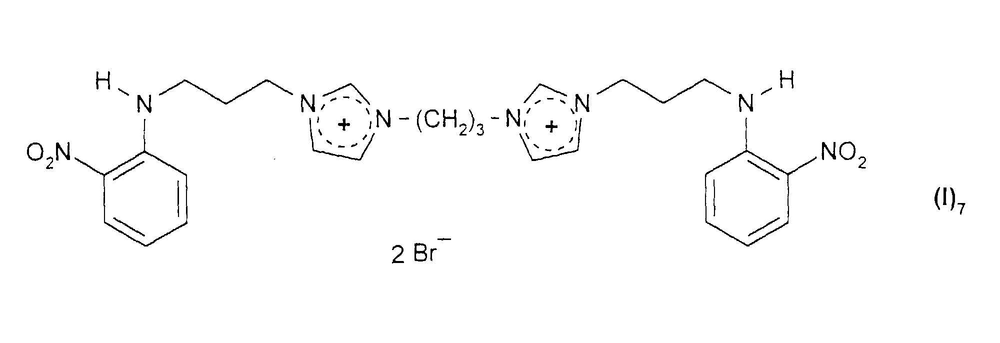

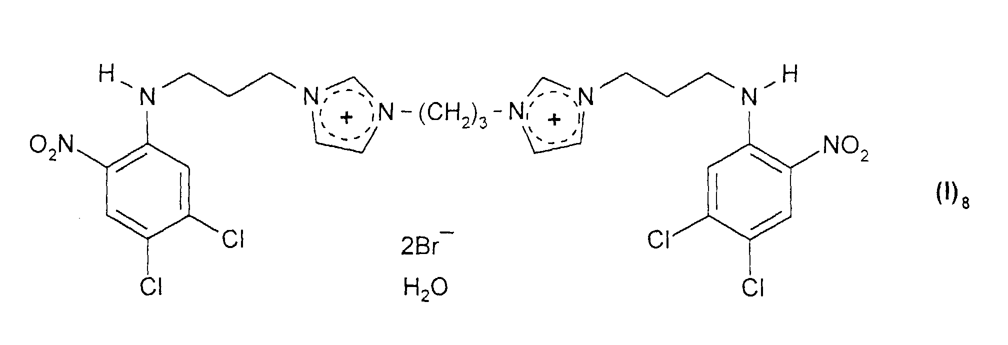

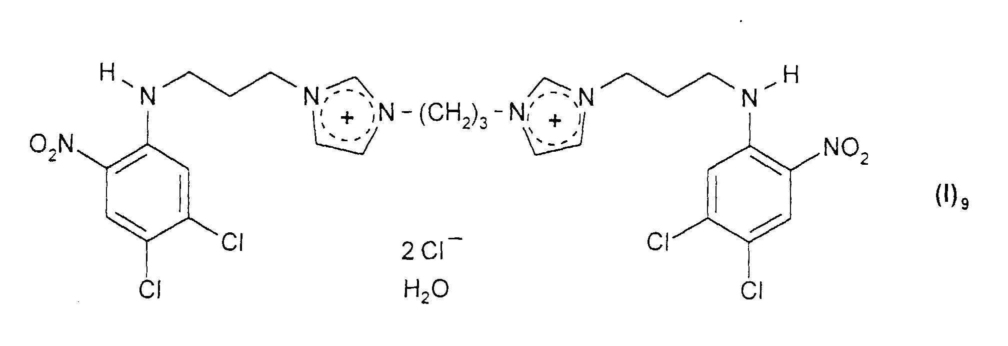

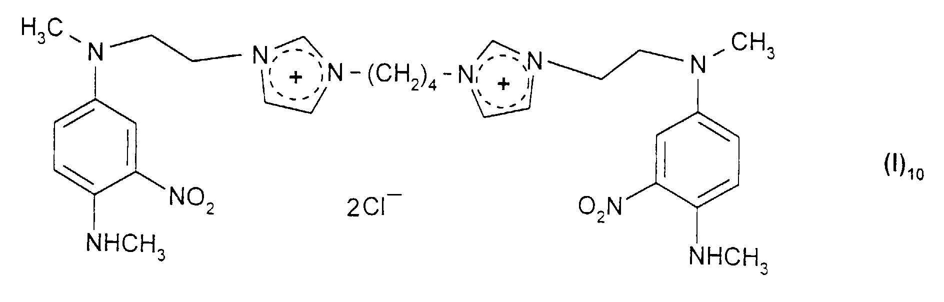

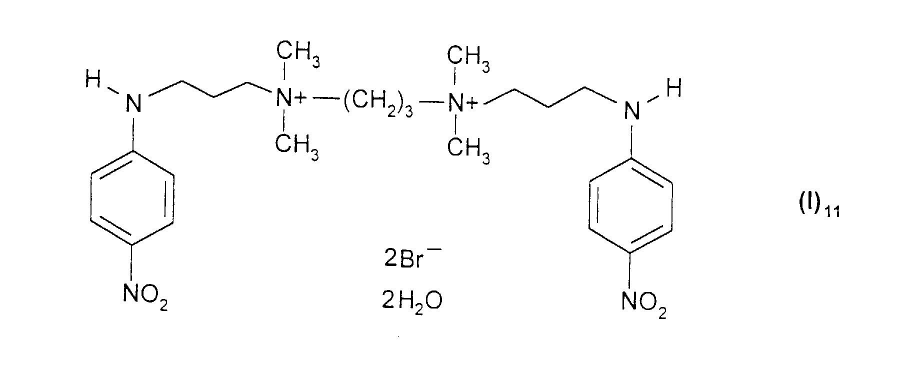

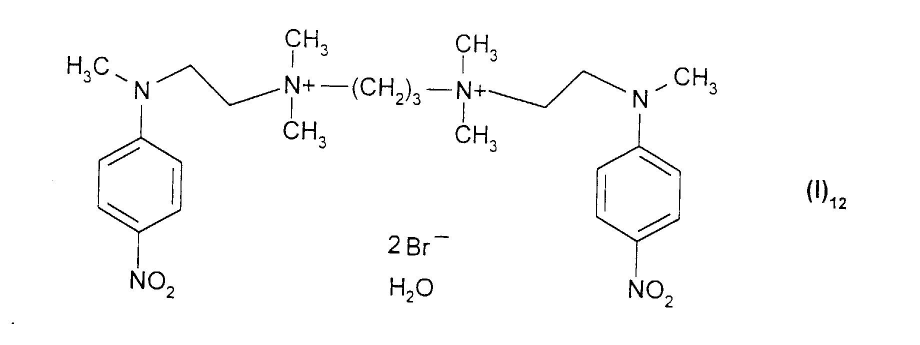

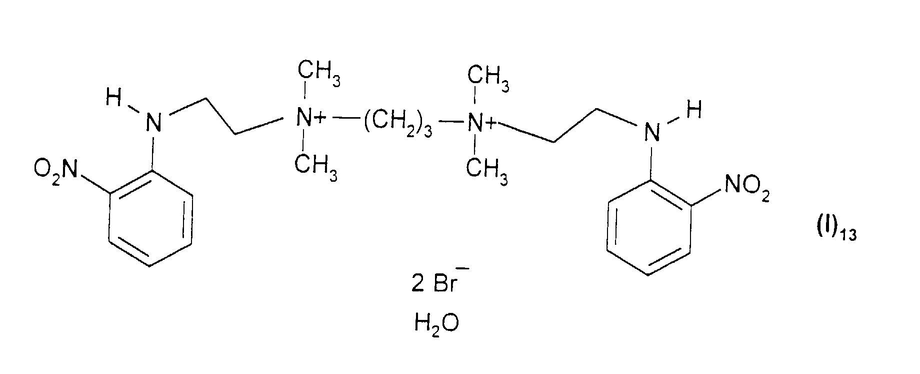

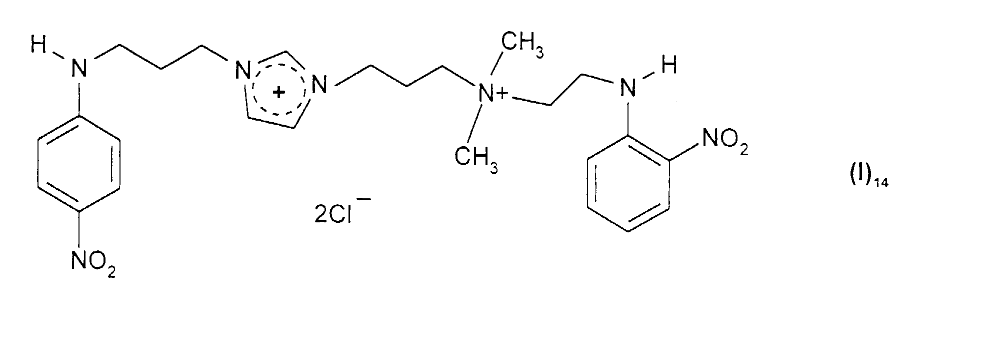

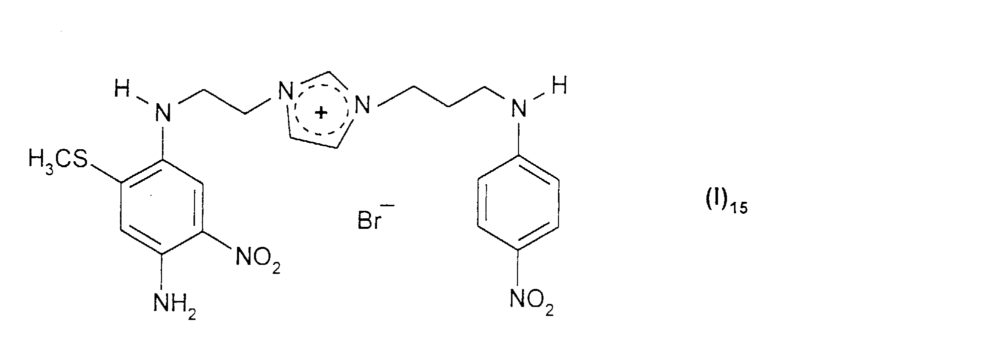

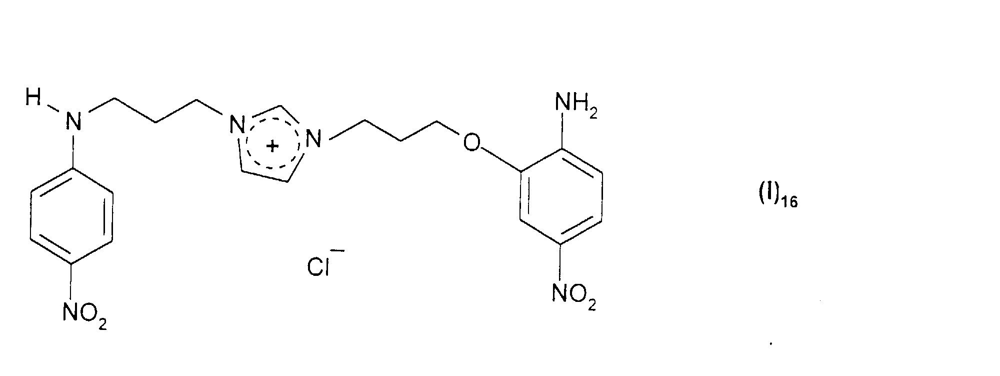

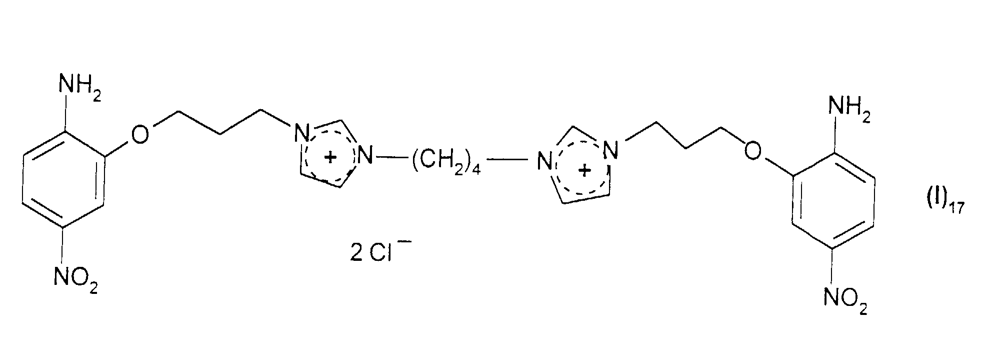

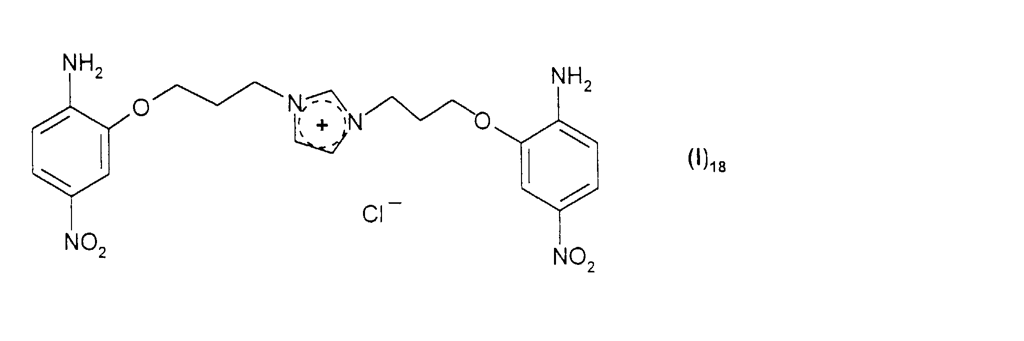

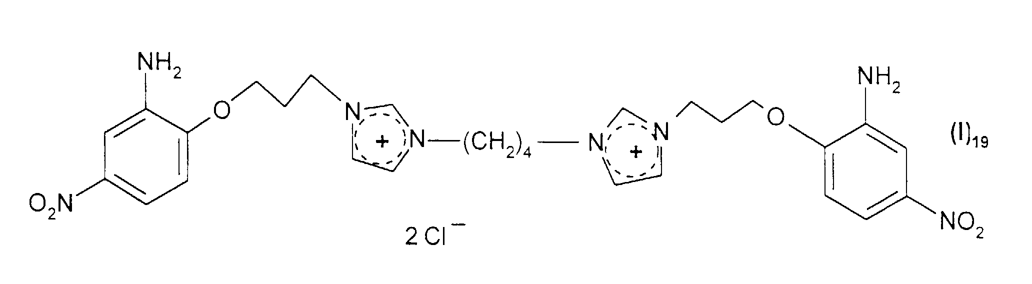

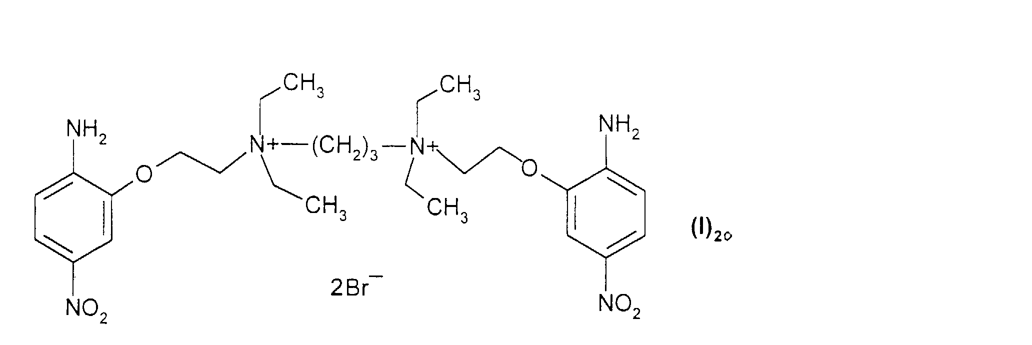

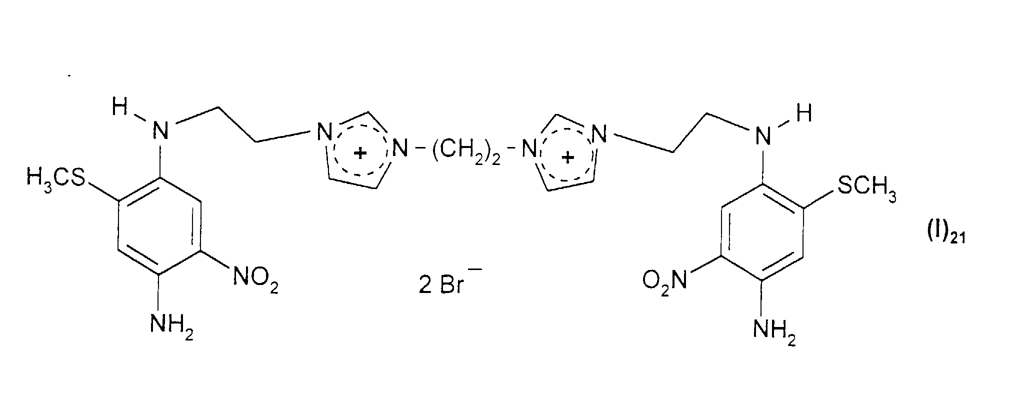

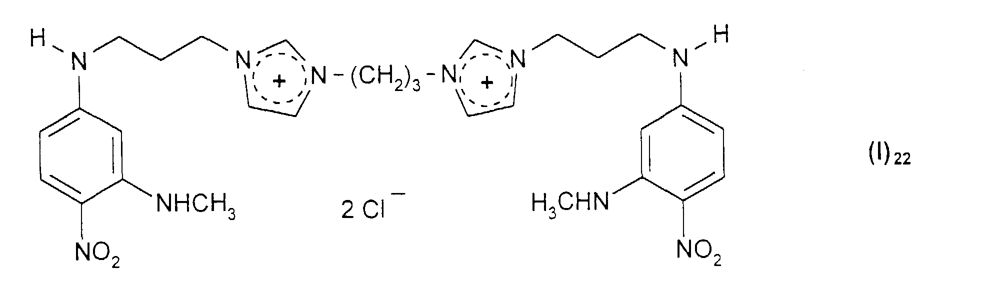

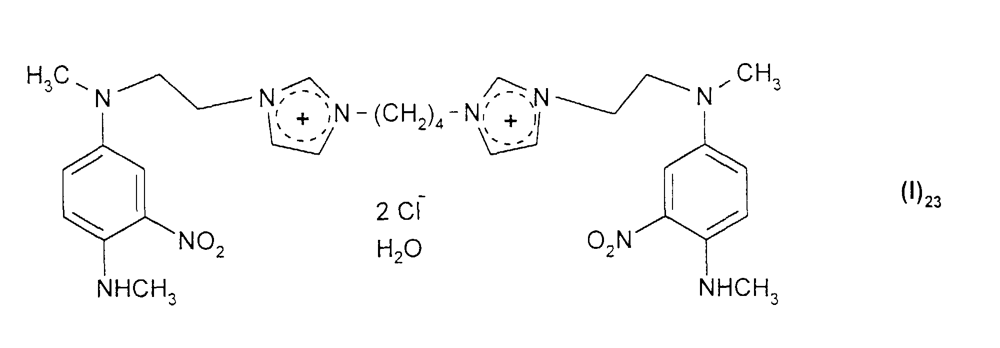

Les composés de formule (I) conformes à l'invention peuvent être facilement obtenus, selon des procédés généralement bien connus de l'état de la technique et notamment par exemple par :
- condensation de deux molécules d'un nitrobenzène porteur d'un radical halogénoalkyle sur une molécule d'un composé porteur de deux radicaux amine tertiaire séparés par un bras de liaison B tel que défini dans la formule (I) décrite ci-dessus, ou bien par
- condensation de deux molécules d'un nitrobenzène porteur d'un radical amine tertiaire sur une molécule d'un composé porteur de deux radicaux halogène séparés par un bras de liaison B tel que défini dans la formule (I) décrite ci-dessus, ou bien,
- (a) condensation d'une molécule d'un nitrobenzène porteur d'un radical amine tertiaire sur une molécule d'un composé porteur de deux radicaux halogène séparés par un bras de liaison B tel que défini dans la formule (I) décrite ci-dessus, et (b) condensation d'une deuxième molécule d'un nitrobenzène différent du premier et porteur lui aussi d'un radical amine tertiaire, ou bien,
- (a) condensation d'une molécule d'un nitrobenzène porteur d'un radical halogénoalkyle sur une molécule d'un composé porteur de deux radicaux amine tertiaire séparés par un bras de liaison B tel que défini dans la formule (I) décrite ci-dessus, et (b) condensation d'une deuxième molécule d'un nitrobenzène différent du premier et porteur lui aussi d'un radical halogénoalkyle, ou bien, -condensation d'une molécule d'un nitrobenzène porteur d'un radical amine tertiaire sur une molécule d'un nitrobenzène porteur d'un radical halogénoalkyle.

L'étape de quaternisation est, généralement par commodité, la dernière étape de la synthèse, mais peut intervenir plus tôt dans la suite des réactions conduisant à la préparation des composés de formule (I).

L'invention a également pour objet une composition de teinture des matières kératiniques, caractérisée par le fait qu'elle comprend, dans un milieu approprié pour la teinture, une quantité efficace d'au moins un composé de formule (I) conforme à l'invention.

L'invention a aussi pour objet une composition pour la teinture directe des fibres kératiniques humaines telles que les cheveux, caractérisée par le fait qu'elle comprend, dans un milieu approprié pour la teinture, une quantité efficace d'au moins un composé de formule (I) conforme à l'invention.

Le ou les composés nitrés di-benzéniques cationiques de formule (I) conformes à l'invention et/ou le ou leurs sels d'addition avec un acide représentent de préférence de 0,005 à 12 % en poids environ du poids total de la composition tinctoriale, et encore plus préférentiellement de 0,05 à 6 % en poids environ de ce poids.

Les composés de formule (I) conformes à l'invention peuvent également servir, dans les procédés bien connus de teinture d'oxydation, utilisant des colorants d'oxydation (précurseurs de colorants d'oxydation et éventuellement des coupleurs), à nuancer ou enrichir de reflets les teintures obtenues avec les colorants d'oxydation.

La composition tinctoriale selon l'invention peut encore contenir, pour élargir la palette de nuances et obtenir des teintes variées, outre les composés nitrés di-benzéniques cationiques de formule (I) selon l'invention, d'autres colorants directs classiquement utilisés, et notamment, des colorants nitrés benzéniques, comme les nitrophénylènediamines, les nitrodiphénylamines, les nitroanilines, les éthers de phénol nitrés ou les nitrophénols, des nitropyridines, des colorants anthraquinoniques, des colorants mono- ou di-azoïques, triarylméthaniques, aziniques, acridiniques et xanthéniques, ou encore des colorants métallifères.

La proportion de tous ces autres colorants directs d'addition peut varier entre 0,5 et 10% en poids environ par rapport au poids total de la composition tinctoriale.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

On peut également ajouter à la composition selon l'invention des amides gras tels que les mono- et di-éthanolamides des acides dérivés du coprah, de l'acide laurique ou de l'acide oléïque, à des concentrations comprises entre environ 0,05 et 10% en poids.

On peut encore ajouter à la composition selon l'invention des agents tensio-actifs bien connus de l'état de la technique et de type anionique, cationique, non-ionique, amphotère, zwittérionique ou leurs mélanges, de préférence en une proportion comprise entre environ 0,1 et 50% en poids et avantageusement entre environ 1 et 20% en poids par rapport au poids total de la composition.
On peut également utiliser des agents épaississants dans une proportion allant d'environ 0,2 à 5%.
Ladite composition tinctoriale peut contenir en outre divers adjuvants usuels tels que des agents anti-oxydants, des parfums, des agents séquestrants, des agents dispersants, des agents de conditionnement du cheveu, des agents conservateurs, des agents opacifiants, ainsi que tout autre adjuvant utilisé habituellement en teinture des matières kératiniques.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 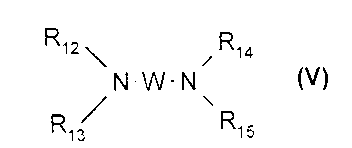 dans laquelle W est un reste propylène éventuellement substitué par un groupement hydroxyle ou un radical alkyle en C₁-C₆ ; R₁₂, R₁₃, R₁₄ et R₁₅, identiques ou différents, représentent un atome d'hydrogène, un radical alkyle en C₁-C₆ ou hydroxyalkyle en C₁-C₆.

La composition tinctoriale selon l'invention peut se présenter sous des formes diverses, telles que sous forme de liquides, de crèmes, de gels, ou sous toute autre forme appropriée pour réaliser une teinture des matières kératiniques, et notamment des cheveux humains. En particulier, elle peut être conditionnée sous pression en flacon aérosol en présence d'un agent propulseur et former une mousse.

Un autre objet de l'invention porte sur un procédé de teinture des fibres kératiniques et en particulier des fibres kératiniques humaines telles que les cheveux, par coloration directe, consistant à laisser agir une composition renfermant au moins un composé nitré-di-benzénique cationique de formule (I) sur les fibres kératiniques sèches ou humides.
On peut utiliser la composition selon l'invention en tant que composition non rincée, c'est-à-dire qu'après application de la composition sur les fibres, on sèche sans rinçage intermédiaire.
Dans les autres modes d'application, après application de la composition sur les fibres pendant un temps de pose variant entre 3 et 60 minutes environ, de préférence entre 5 et 45 minutes environ, on rince, éventuellement on lave, puis on rince à nouveau, et on sèche.

Des exemples concrets et non limitatifs illustrant l'invention vont maintenant être donnés.

### EXEMPLES DE PREPARATION

### Exemple 1 : Préparation du composé de formule (I)₁

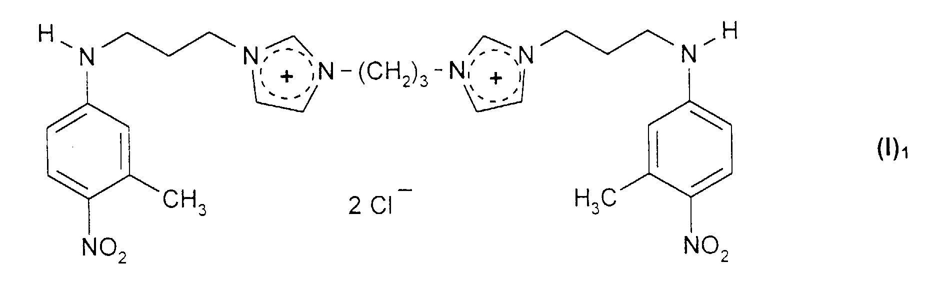

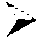 **1**^{**ère**} **étape**:

### synthèse du (3-imidazol-1-yl-propyl)-(3-méthyl-4-nitrophényl)-amine

On a chauffé à 90°C un mélange de 125,2g (1 mole) de 3-imidazol-1-yl-propylamine (RN 5036-48-6) et 41,4g (0,3 mole) de carbonate de potassium dans 140ml d'eau.
On a ajouté goutte à goutte 77,6g (0,5 mole) de 4-fluoro-2-méthyl-1-nitrobenzène (RN 446-33-3) en 45 minutes et maintenu à 90-95°C pendant 2 heures. On a refroidi dans un bain de glace, essoré le précipité cristallisé, lavé à l'eau et séché à 40°C sous vide et sur anhydride phosphorique.
Après recristallisation de l'éthanol absolu au reflux, on a obtenu 96g de cristaux jaunes fondant à 133°C (Kofler) et dont l'analyse élémentaire calculée pour C₁₃H₁₆N₄O₂ était :

| % | C | H | N | O |
|---|---|---|---|---|
| Calculé : | 59,99 | 6,20 | 21,52 | 12,29 |
| Trouvé: | 59,55 | 6,22 | 21,43 | 12,88 |

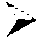 **2**^{**ème**} **étape**:

### quaternisation

On a chauffé 6 heures au reflux un mélange de 88,9g (0,341 mole) de (3-imidazol-1-yl-propyl)-(3-méthyl-4-nitro-phényl)-amine obtenu à l'étape précédente et de 19,3g (0,1705 mole) de 1,3-dichloro-propane (RN 142-28-9) dans 220ml de pentanol normal. Le milieu réactionnel était une solution.
On a refroidi dans un bain de glace : une gomme a précipité puis cristallisé en masse. On a essoré, lavé avec de l'éthanol absolu, recristallisé de l'éthanol au reflux et séché à 40°C sous vide.
On a obtenu 56g de cristaux jaunes fondant à 138-140°C (Kofler) et dont l'analyse élémentaire calculée pour C₂₉H₃₆N₉O₄Cl₂ + H₂O était:

| **%** | **C** | **H** | **N** | **O** | **Cl** |
|---|---|---|---|---|---|
| Calculé : | 53,46 | 6,19 | 17,20 | 12,28 | 10,88 |
| Trouvé : | 52,69 | 6,25 | 17,06 | 12,89 | 10,99 |

### Exemple 2 : Préparation du composé de formule (I)₂

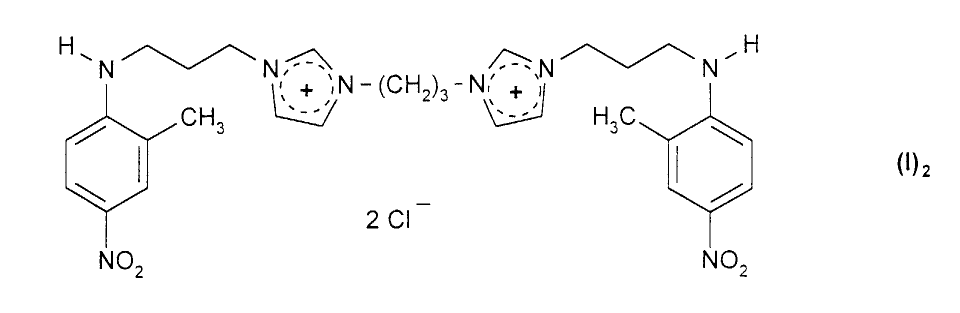

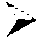 **1**^{**ère**} **étape :**

### synthèse du (3-imidazol-1-yl-propyl)-(2-méthyl-4-nitrophényl)-amine

On a chauffé à 90°C un mélange de 250,4g (2 moles) de 3-imidazol-1-yl-propylamine (RN 5036-48-6) et 82,8g (0,6 mole) de carbonate de potassium dans 280ml d'eau.
On a ajouté goutte à goutte 155,1g (1 mole) de 1-fluoro-2-méthyl-4-nitro-benzène (RN 455-88-9) en 30 minutes et on a maintenu à 90-95°C pendant 4 heures. On a refroidi dans un bain de glace, essoré le précipité cristallisé, lavé à l'isopropanol et séché à 40°C sous vide.
Après recristallisation de l'éthanol au reflux on a obtenu 144,8g de cristaux orangés fondant à 163°C (Kofler) et dont l'analyse élémentaire calculée pour C₁₃H₁₆N₄O₂ était :

| **%** | **C** | **H** | **N** | **O** |
|---|---|---|---|---|
| Calculé : | 59,99 | 6,20 | 21,52 | 12,29 |
| Trouvé: | 59,60 | 6,15 | 21,46 | 13,12 |

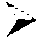 **2**^{**ème**} **étape**:

### quaternisation

On a chauffé 6 heures au reflux un mélange de 130,1g (0,5 mole) de (3-imidazol-1-yl-propyl)-(2-méthyl-4-nitro-phényl)-amine obtenu à l'étape précédente et de 28,25g (0,25 mole) de 1,3-dichloro-propane (RN 142-28-9) dans 320ml de pentanol normal.
Le milieu réactionnel était une solution.
On a refroidi dans un bain de glace et ajouté de l'éthanol absolu : une gomme a précipité puis cristallisé.
On a essoré, lavé avec de l'éthanol absolu et séché à 40°C sous vide.
On a obtenu 129,6g de cristaux jaunes fondant à 148-150°C (Kofler) et dont l'analyse élémentaire calculée pour C₂₉H₃₈N₈O₄Cl₂ + 2,5H₂O était :

| **%** | **C** | **H** | **N** | **O** | **Cl** |
|---|---|---|---|---|---|
| Calculé : | 51,33 | 6,39 | 16,51 | 15,32 | 10,45 |
| Trouvé : | 51,69 | 6,45 | 16,62 | 15,38 | 10,33 |

### Exemple 3 : Préparation du composé de formule (I)₃

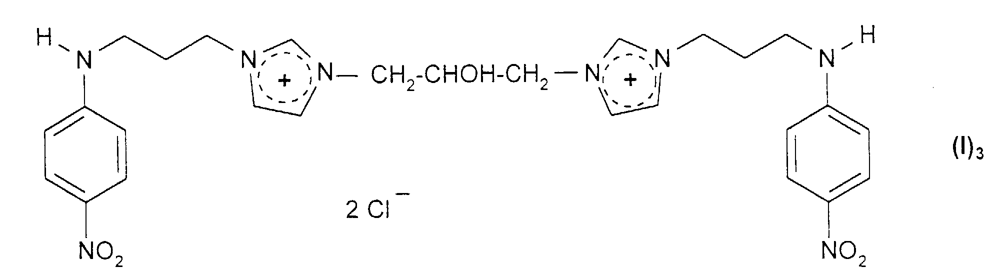

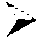 **1**^{**ère**} **étape**:

### synthèse du (3-imidazol-1-yl-propyl)-(4-nitro-phényl)-amine

On a chauffé au bain-marie bouillant un mélange de 150,2g (1,2 moles) de 3-imidazol-1-yl-propylamine (RN 5036-48-6) et 139,4ml (1 mole) de triéthylamine dans 200ml de dioxane.
On a ajouté goutte à goutte 141,1g (1 mole) de 1-fluoro-4-nitro-benzène (RN 350-46-9) en 30 minutes et maintenu à 90-95°C pendant 1 heure.
On a versé dans 2kg d'eau glacée, essoré le précipité cristallisé, lavé à l'eau et recristallisé de l'éthanol au reflux.
On a obtenu 106,0g de cristaux jaunes fondant à 126°C (Kofler) et dont l'analyse élémentaire calculée pour C₁₂H₁₄N₄O₂ était:

| **%** | **C** | **H** | **N** | **O** |
|---|---|---|---|---|
| Calculé : | 58,53 | 5,73 | 22,75 | 12,99 |
| Trouvé : | 58,33 | 5,83 | 22,81 | 13,41 |

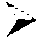 **2**^{**ème**} **étape**:

### quaternisation

On a chauffé 8 heures au reflux un mélange de 24,6g (0,1 mole) de (3-imidazol-1-yl-propyl)-(4-nitro-phényl)-amine obtenu à l'étape précédente et de 6,45g (0,05 mole) de 1,3-dichloro-propan-2-ol (RN 96-23-1) dans 100ml de toluène. Une gomme en suspension a cristallisé.
On a refroidi, essoré le précipité cristallisé, réempâté deux fois dans le minimum d'éthanol absolu et séché à 45°C sous vide.
On a obtenu 24,8g de cristaux jaunes fondant à 228-230°C (Kofler) et dont l'analyse élémentaire calculée pour C₂₇H₃₄N₈O₅Cl₂ était :

| **%** | **C** | **H** | **N** | **O** | **Cl** |
|---|---|---|---|---|---|
| Calculé : | 52,18 | 5,51 | 18,03 | 12,87 | 11,41 |
| Trouvé : | 52,23 | 5,55 | 18,03 | 12,80 | 11,44 |

### Exemple 4 : Préparation du composé de formule (I)₄

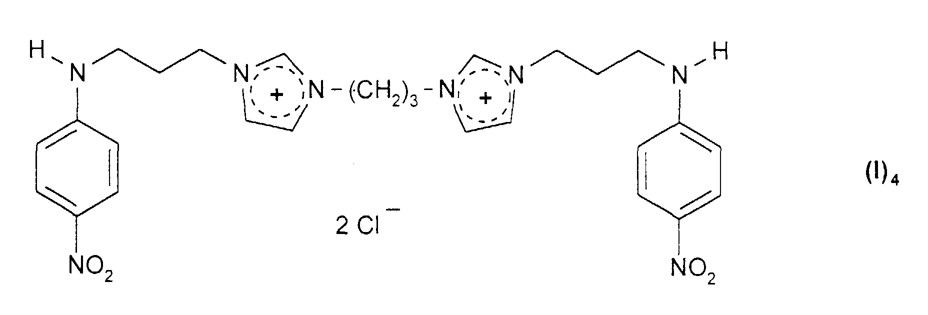

On a utilisé le mode opératoire décrit pour l'exemple 3, 2^{ème} étape.
A partir de 39,4g (0,16 mole) de (3-imidazol-1-yl-propyl)-(4-nitro-phényl)-amine obtenu à l'étape 1 de l'exemple 3 et de 9,03g (0,08 mole) de 1,3-dichloro-propane (RN 142-28-9) on a obtenu 23,3g de cristaux jaunes fondant à 186°C (Kofler) et dont l'analyse élémentaire calculée pour C₂₇H₃₄N₈O₄Cl₂ + 1/3 H₂O était :

| **%** | **C** | **H** | **N** | **O** | **Cl** |
|---|---|---|---|---|---|
| Calculé: | 53,03 | 5,71 | 18,32 | 11,34 | 11,59 |
| Trouvé: | 53,00 | 5,68 | 18,33 | 11,19 | 11,44 |

### Exemple 5 : Préparation du composé de formule (I)₅

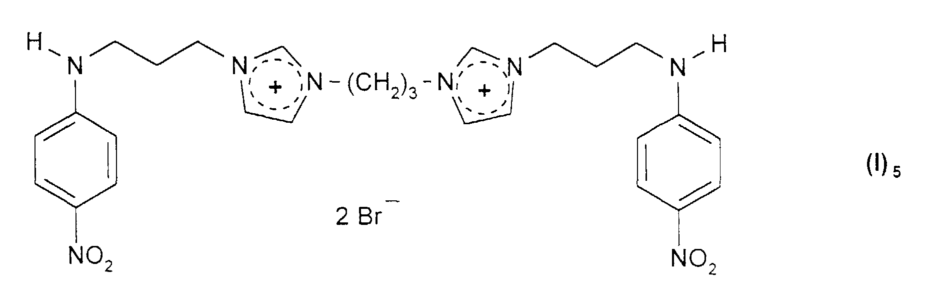

On a utilisé le mode opératoire décrit pour l'exemple 3, 2^{ème} étape.
A partir de 14,8g (0,06 mole) de (3-imidazol-1-yl-propyl)-(4-nitro-phényl)-amine obtenu à l'étape 1 de l'exemple 3 et de 6,05g (0,03 mole) de 1,3-dibromo-propane (RN 109-64-8) on a obtenu 19,2g de cristaux jaunes fondant à 203°C (Kofler) et dont l'analyse élémentaire calculée pour C₂₇H₃₄N₈O₄Br₂ était :

| **%** | **C** | **H** | **N** | **O** | **Br** |
|---|---|---|---|---|---|
| Calculé: | 46,70 | 4,94 | 16,14 | 9,22 | 23,01 |
| Trouvé: | 46,56 | 5,03 | 16,21 | 9,36 | 22,70 |

### Exemple 6 : Préparation du composé de formule (I)₅

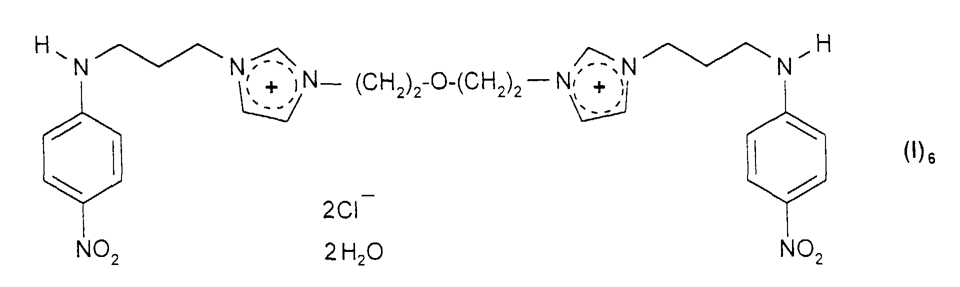

On a utilisé le mode opératoire décrit pour l'exemple 3, 2^{ème} étape.
A partir de 24,6g (0,10 mole) de (3-imidazol-1-yl-propyl)-(4-nitro-phényl)-amine obtenu à l'étape 1 de l'exemple 3 et de 7,15g (0,05 mole) de 1-chloro-2-(2-chloroéthoxy)-ethane (RN 111-44-4) on a obtenu 21,6g de cristaux jaunes fondant à 118-120°C (Kofler) et dont l'analyse élémentaire calculée pour C₂₈H₃₆N₈O₅Cl₂ + 2H₂O était :

| **%** | **C** | **H** | **N** | **O** | **Cl** |
|---|---|---|---|---|---|
| Calculé : | 50,08 | 6,00 | 16,68 | 16,68 | 10,56 |
| Trouvé: | 50,76 | 6,08 | 16,38 | 16,34 | 10,34 |

### Exemple 7 : Préparation du composé de formule (I)₇

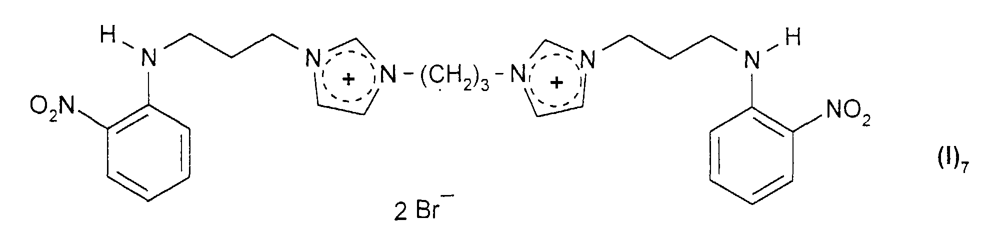

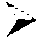 **1**^{**ère**} **étape:**

### synthèse du (3-imidazol-1-yl-propyl)-(2-nitro-phényl)-amine

On a chauffé au bain-marie bouillant un mélange de 187,8g (1,5 moles) de 3-imidazol-1-yl-propylamine (RN 5036-48-6) et 82,8g (0,6mole) de carbonate de potassium dans 280ml d'eau.
On a ajouté goutte à goutte 141,1g (1 mole) de 1-fluoro-2-nitro-benzène (RN 1493-27-2) en 50 minutes et maintenu à 90-95°C pendant 2 heures.
On a versé dans 2kg d'eau glacée, essoré le précipité cristallisé, lavé à l'eau et recristallisé de l'alcool isopropylique au reflux.
On a obtenu 109,3g de cristaux jaunes fondant à 80°C (Kofler) et dont l'analyse élémentaire calculée pour C₁₂H₁₄N₄O₂ était :

| **%** | **C** | **H** | **N** | **O** |
|---|---|---|---|---|
| Calculé : | 58,53 | 5,73 | 22,75 | 12,99 |
| Trouvé: | 58,62 | 5,78 | 22,54 | 13,07 |

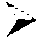 **2**^{**ème**} **étape**:

### quaternisation

On a utilisé le mode opératoire décrit pour l'exemple 3, 2ème étape, en prenant pour solvant le méthyl-2-propanol-1 à la place du toluène.
A partir de 74,0g (0,3 mole) de (3-imidazol-1-yl-propyl)-(2-nitro-phényl)-amine obtenu à l'étape précédente et de 30,3g (0,15 mole) de 1,3-dibromo-propane (RN 109-64-8) on a obtenu, après recristallisation de l'éthanol au reflux, 86,4g de cristaux orangés fondant à 166°C (Kofler) et dont l'analyse élémentaire calculée pour C₂₇H₃₄N₆O₄Br₂ était :

| **%** | **C** | **H** | **N** | **O** | **Br** |
|---|---|---|---|---|---|
| Calculé: | 46,70 | 4,94 | 16,14 | 9,22 | 23,01 |
| Trouvé: | 46,59 | 5,00 | 16,15 | 9,41 | 22,97 |

### Exemple 8 : Préparation du composé de formule (I)₈

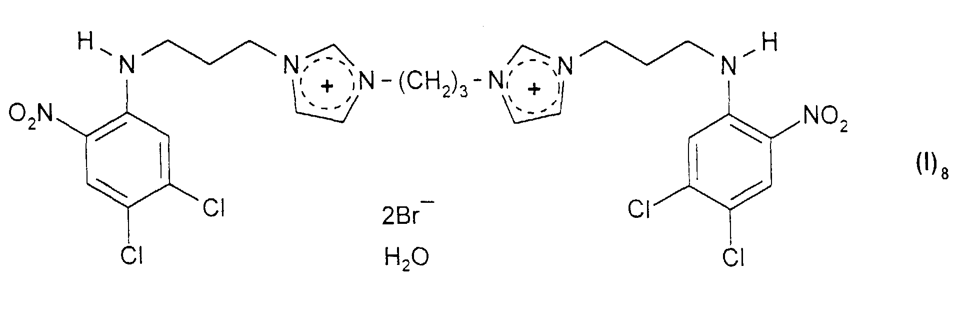

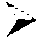 **1**^{**ère**} **étape**:

### synthèse du (4,5-dichloro-2-nitro-phényl)-(3-imidazol-1-yl-propyl)-amine

On a chauffé au reflux un mélange de 50,1g (0,45 mole) de 3-imidazol-1-yl-propylamine (RN 5036-48-6) et 62,7ml (0,45 mole) de triéthylamine dans 100ml de diméthoxy-1,2 éthane.
On a ajouté par portions 90,6g (0,4 mole) de 1,2,4-trichloro-5-nitro-benzène (RN 89-69-0) en 30 minutes et maintenu au reflux pendant 5 heures.
On a versé sur 1,5kg d'eau glacée, essoré le précipité cristallisé, lavé à l'eau et séché à 40°C sous vide et sur anhydride phosphorique.
Après recristallisation de l'éthanol au reflux on a obtienu 36g de cristaux jaune orangé fondant à 130°C (Kofler) et dont l'analyse élémentaire calculée pour C₁₂H₁₂N₄O₂Cl₂ était :

| **%** | **C** | **H** | **N** | **O** | **Cl** |
|---|---|---|---|---|---|
| Calculé : | 45,73 | 3,84 | 17,78 | 10,15 | 22,50 |
| Trouvé : | 45,61 | 3,88 | 17,69 | 10,09 | 22,43 |

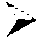 **2**^{**ème**} **étape** :

### quaternisation

On a utilisé le mode opératoire décrit pour l'exemple 3, 2ème étape, en prenant pour solvant le méthyl-2-propanol-1 à la place du toluène.
A partir de 47,27g (0,15 mole) de (4,5-dichloro-2-nitro-phényl)-(3-imidazol-1-yl-propyl)-amine obtenu à l'étape précédente et de 15,15g (0,075 mole) de 1,3-dibromo-propane (RN 109-64-8) on a obtenu, après recristallisation d'un mélange d'éthanol et d'eau au reflux, 49,0g de cristaux orangés fondant vers 123-125°C (Kofler) et dont l'analyse élémentaire calculée pour C₂₇H₃₀N₈O₄Cl₄Br₂ + H₂O était :

| **%** | **C** | **H** | **N** | **O** | **Cl** | **Br** |
|---|---|---|---|---|---|---|
| Calculé : | 38,14 | 3,79 | 13,18 | 9,41 | 16,68 | 18,8 |
| Trouvé : | 37,90 | 3,81 | 13,15 | 9,36 | 16,79 | 19,25 |

### Exemple 9 : Préparation du composé de formule (I)₉

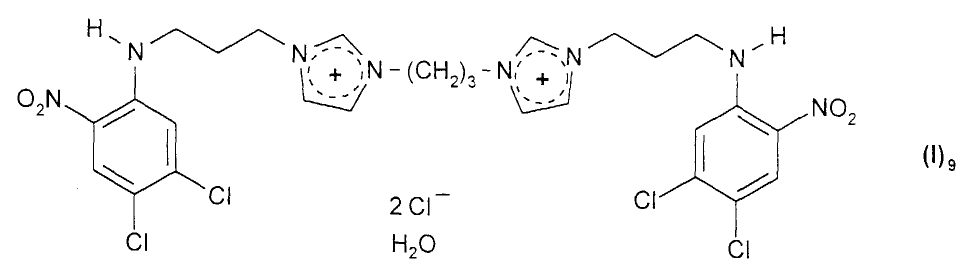

### quaternisation

A partir de 94,5g (0,3 mole) de (4,5-dichloro-2-nitro-phényl)-(3-imidazol-1-yl-propyl)-amine obtenu à la première étape de l'exemple 8 et de 16,95g (0,15 mole) de 1,3-dichloro-propane (RN 142-28-9) chauffés 19 heures à 150°C dans le pentanol normal, on a obtenu, après recristallisation de l'éthanol au reflux, 38,3g de cristaux orangés fondant à 224°C (Kofler) et dont l'analyse élémentaire calculée pour C₂₇H₃₀N₈O₄Cl₆ + ½ H₂O était :

| **%** | **C** | **H** | **N** | **O** | **Cl** |
|---|---|---|---|---|---|
| Calculé : | 43,11 | 4,15 | 14,89 | 9,57 | 28,28 |
| Trouvé : | 43,38 | 4,25 | 14,71 | 9,78 | 28,57 |

### Exemple 10 : Préparation du composé de formule (I)₁₀

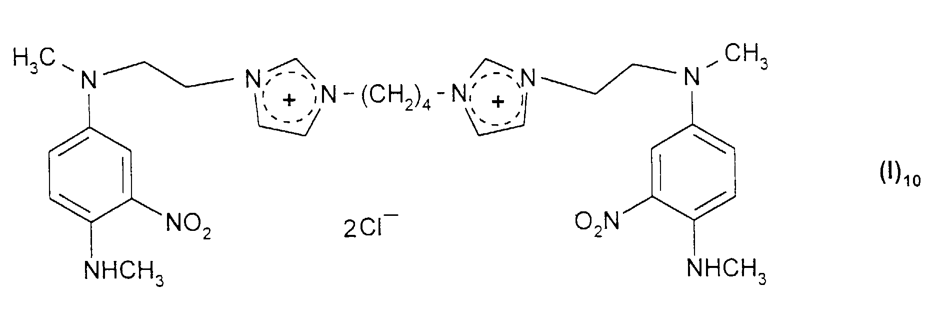

On a chauffé au reflux de l'isobutanol pendant 20 heures un mélange de 24,1g (0,1 mole) de N4-(2-chloro-éthyl)-N1,N4-diméthyl-2-nitro-benzène-1,4-diamine (RN 14607-54-6) et de 10,2g (0,05 mole) de 1,4-di-imidazol-1-yl-butane (RN 69506-86-1) dans 50ml d'isobutanol.
On a essoré à température ambiante le précipité cristallisé formé.
Après purification par recristallisation de l'éthanol au reflux, on a obtenu 20,3g de cristaux violet foncé fondant vers 175-177°C (Kofler) et dont l'analyse élémentaire calculée pour C₃₀H₄₂N₁₀O₄Cl₂ + H₂O était :

| **%** | **C** | **H** | **N** | **O** | **Cl** |
|---|---|---|---|---|---|
| Calculé: | 51,80 | 6,38 | 20,13 | 11,50 | 10,19 |
| Trouvé : | 50,79 | 6,43 | 19,39 | 11,50 | 10,20 |

### Exemple 11 : Préparation du composé de formule (I)₁₁

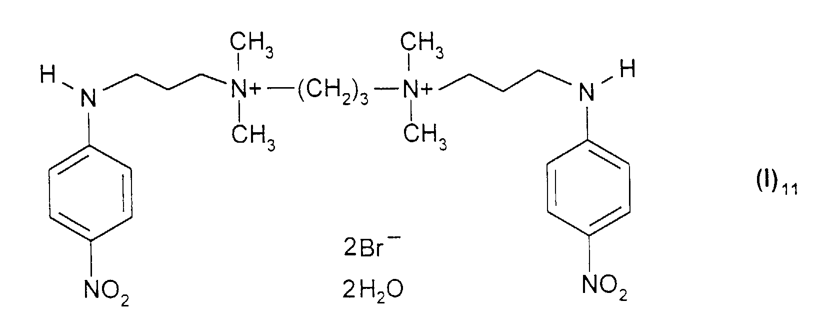

On a utilisé le mode opératoire décrit pour l'exemple 3, 2^{ème} étape.
A partir de 44,6g (0,2 mole) de N,N-diméthyl-N'-(4-nitro-phényl)-propane-1,3-diamine (RN 25238-54-4) et de 20,2g (0,1 mole) de 1,3-dibromo-propane (RN 109-64-8) on a obtenu 47,8g de cristaux jaunes fondant avec décomposition vers 230°C (Kofler) et dont l'analyse élémentaire calculée pour C₂₅H₄₀N₆O₄Br₂ + H₂O était :

| **%** | **C** | **H** | **N** | **O** | **Br** |
|---|---|---|---|---|---|
| Calculé : | 45,06 | 6,35 | 12,61 | 12,00 | 23,98 |
| Trouvé: | 45,15 | 6,35 | 12,36 | 11,61 | 24,02 |

### Exemple 12 : Préparation du composé de formule (I)₁₂

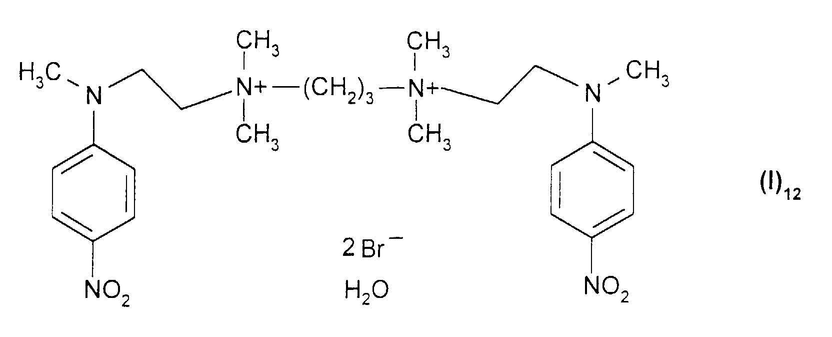

On a utilisé le mode opératoire décrit pour l'exemple 3, 2^{ème} étape, mais en prenant pour solvant le méthyl-2 propanol-1 à la place du toluène.
A partir de 53,6g (0,24 mole) de N,N,N'-triméthyl-N'-(4-nitro-phényl)-éthane-1,2-diamine (RN 176665-67-1) et de 24,2g (0,12 mole) de 1,3-dibromo-propane (RN 109-64-8) on a obtenu 63,5g de cristaux jaunes fondant vers 147°C (Kofler) et dont l'analyse élémentaire calculée pour C₂₅H₄₀N₆O₄Br₂ + H₂O était :

| % | C | H | N | O | Br |
|---|---|---|---|---|---|
| Calculé : | 45,06 | 6,35 | 12,61 | 12,00 | 23,98 |
| Trouvé: | 45,04 | 6,38 | 12,60 | 12,68 | 23,98 |

### Exemple 13 : Préparation du composé de formule (I)₁₃

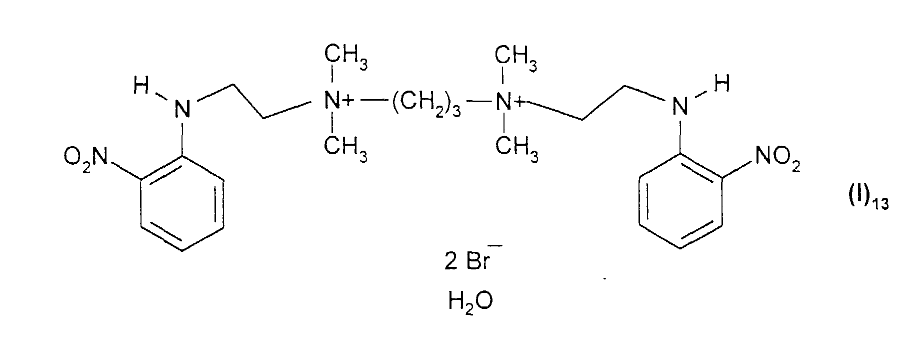

On a utilisé le mode opératoire décrit pour l'exemple 3.
A partir de 62,7g (0,3 mole) de N,N-diméthyl-N'-(2-nitro-phényl)-éthane-1,2-diamine (RN 25238-55-5) et de 30,3g (0,15 mole) de 1,3-dibromo-propane (RN 109-64-8) on a obtenu, après recristallisation d'un mélange d'éthanol et d'eau au reflux, 67,0g de cristaux jaunes fondant à 220°C (Kofler) et dont l'analyse élémentaire calculée pour C₂₃H₃₆N₆O₄Br₂ + H₂O était :

| **%** | **C** | **H** | **N** | **O** | **Br** |
|---|---|---|---|---|---|
| Calculé : | 43,27 | 6,00 | 13,16 | 12,53 | 25,03 |
| Trouvé : | 43,24 | 6,14 | 12,59 | 12,72 | 24,74 |

### EXEMPLES DE COMPOSITIONS TINCTORIALES

### EXEMPLES 1 à 6 :

On a préparé les 6 compositions de teinture directe réunies dans le tableau suivant *(toutes teneurs exprimées en grammes - M.A. désigne Matière Active):*

| | Exemple 1 | Exemple 2 | Exemple 3 | Exemple 4 | Exemple 5 | Exemple 6 |
|---|---|---|---|---|---|---|
| colorant de formule (I)₃ | 0,311 | | | | | |
| colorant de formule (I)₆ | | 0,318 | | | | |
| colorant de formule (I)₄ | | | 0,303 | | | |
| colorant de formule (I)₁₁ | | | | 0,333 | | |
| colorant de formule (I)₇ | | | | | 0,347 | |
| colorant de formule (I)₁₃ | | | | | | 0,319 |
| Hydroxyéthylcellulose vendue sous la dénomination NATROSOL 250 MR par la société Aqualon | 0,72 | 0,72 | 0,72 | 0,72 | 0,72 | 0,72 |
| Alcool benzylique | 4 | 4 | 4 | 4 | 4 | 4 |
| Polyéthylèneglycol à 6 oxyde d'éthylène | 6 | 6 | 6 | 6 | 6 | 6 |
| Alkyl(C8-C10) polyglucoside en solution aqueuse à 60% M.A. vendu sous la dénomination ORAMIX CG 110 par la société Seppic | 4,5 M.A. | 4,5 M.A. | 4,5 M.A. | 4,5 M.A. | 4,5 M.A. | 4,5 M.A. |
| Tampon phosphate pH 7 q.s.p | 100 | 100 | | 100 | 100 | |
| Tampon phosphate pH 9 (acide borique/chlorure de potassium/hydroxyde de sodium q.s.p | | | 100 | | | 100 |

### EXEMPLE 7 :

On a préparé la composition de teinture directe suivante :
*(toutes teneurs exprimées en grammes - M.A. désigne Matière Active)*

| | Exemple 7 |
|---|---|
| colorant de formule (I)₂₃ | 0,696 |
| Hydroxyéthylcellulose vendue sous la dénomination NATROSOL 250 MR par la société Aqualon | 0,384 |
| Alcool benzylique | 4 |
| Polyéthylèneglycol 400 | 6 |
| Alkyl(C8-C10) polyglucoside en solution aqueuse à 60% M.A. vendu sous la dénomination ORAMIX CG 110 par la société Seppic | 5 M.A. |
| Tampon phosphate pH 9 (acide borique/chlorure de potassium/hydroxyde de sodium q.s.p | 100 |

On a appliqué chacune des compositions ci-dessus sur des mèches de cheveux gris naturels ou permanentés à 90% de blancs et on a laissé poser pendant 20 minutes. Après rinçage à l'eau courante et séchage, les cheveux ont été teints dans une nuance qui est exprimée dans le tableau ci-dessous.

| | |
|---|---|
| Composition de l'exemple 1 | Jaune mat intense |
| Composition de l'exemple 2 | Jaune mat intense |
| Composition de l'exemple 3 | Jaune mat intense |
| Composition de l'exemple 4 | Jaune mat intense |
| Composition de l'exemple 5 | Jaune orange intense |
| Composition de l'exemple 6 | Jaune intense |
| Composition de l'exemple 7 | Violet |

## Revendications

1. Utilisation à titre de colorants directs dans des, ou pour la fabrication de, compositions tinctoriales pour matières kératiniques en particulier pour fibres kératiniques humaines et notamment les cheveux, de composés de formule (I) suivante, et leurs sels d'addition avec un acide : 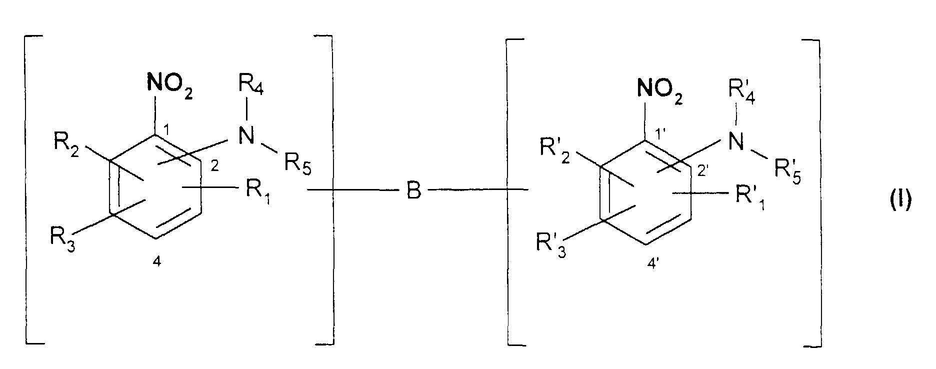 dans laquelle :
• B est un bras de liaison qui représente une chaîne alkyle comportant de préférence de 1 à 14 atomes de carbone, linéaire ou ramifiée pouvant être interrompue par un ou plusieurs groupements Z et/ou par un ou plusieurs hétéroatomes tels que des atomes d'oxygène, de soufre ou d'azote, et éventuellement substituée par un ou plusieurs radicaux hydroxyle ou alcoxy en C₁-C₆, et pouvant porter une ou plusieurs fonctions cétone ;
• R₁, R₂, R₃, R'₁, R'₂ et R'₃, qui peuvent être identiques ou différents, représentent l'une des deux valences d'un bras de liaison B, un atome d'hydrogène ; un atome d'halogène ; un groupement Z ; un radical alkyl(C₁-C₆) carbonyle ; un radical aminoalkyl(C₁-C₆)carbonyle ; un radical N-Z-aminoalkyl(C₁-C₆)carbonyle ; un radical N-alkyl(C₁-C₆)aminoalkyl(C₁-C₆)carbonyle ; un radical N,N-dialkyl(C₁-C₆)aminoalkyl(C₁-C₆)carbonyle; un radical aminoalkyl(C₁-C₆)carbonylalkyle(C₁-C₆); un radical N-Z-aminoalkyl(C₁-C₆)carbonylalkyle(C₁-C₆) ; un radical N-alkyl(C₁-C₆)aminoalkyl(C₁-C₆)carbonylalkyle(C₁-C₆); un radical N,N-dialkyl(C₁-C₆)aminoalkyl(C₁-C₆)carbonylalkyle(C₁-C₆); un radical carboxy ; un radical alkyl(C₁-C₆) carboxy ; un radical alkyl(C₁-C₆) sulfonyle ; un radical aminosulfonyle ; un radical N-Z-aminosulfonyle ; un radical N-alkyl(C₁-C₆)aminosulfonyle ; un radical N,N-dialkyl(C₁-C₆)aminosulfonyle; un radical aminosulfonylalkyle(C₁-C₆); un radical N-Z-aminosulfonylalkyle(C₁-C₆); un radical N-alkyl(C₁-C₆)aminosulfonylalkyle(C₁-C₆); un radical N,N-dialkyl(C₁-C₆)aminosulfonylalkyle(C₁-C₆); un radical carbamyle ; un radical N-alkyl(C₁-C₆)carbamyle ; un radical N,N-dialkyl(C₁-C₆)carbamyle ; un radical carbamylalkyle(C₁-C₆); un radical N-alkyl(C₁-C₆)carbamylalkyle(C₁-C₆); un radical N,N-dialkyl(C₁-C₆)carbamylalkyle(C₁-C₆); un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical trifluoroalkyle en C₁-C₆ ; un radical cyano ; un radical amino substitué ou non substitué, avec les mêmes significations que dans le groupement -NR₄R₅ ou -NR'₄R'₅ décrits ci-dessous, et pouvant être identique ou différent ; un radical aminoalkyle(C₁-C₆) dont l'alkyle est substitué ou non substitué par un ou plusieurs radicaux hydroxy et dont l'amine est substituée par un ou deux radicaux, identiques ou différents, choisis parmi les radicaux alkyle en C₁-C₆, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C,-C₆)carbamyle, alkyl(C₁-C₆)sulfonyle, formyle, trifluoroalkyl(C₁-C₆)carbonyle, alkyl(C₁-C₆)carboxy, thiocarbamyle, ou par un groupement Z ; un groupement OR₆ ou -SR₆,
• R₆ désigne l'une des deux valences d'un bras de liaison B, un atome d'hydrogène ; un radical alkyle en C₁-C₆; un radical monohydroxyalkyle en C₁-C₆ ; un radical polyhydroxyalkyle en C₂-C₆ ; un groupement Z ; un radical alcoxy(C₁-C₆)alkyle en C₁-C₆ ; un radical aryle ; un radical benzyle ; un radical carboxyalkyle en C₁-C₆ ; un radical alkyl(C₁-C₆)carboxyalkyle en C₁-C₆ ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical trifluoroalkyle en C₁-C₆ ; un radical aminosulfonylalkyle en C₁-C₆ ; un radical N-Z-aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical aminoalkyle en (C₁-C₆) ; un radical aminoalkyle en (C₁-C₆) dont l'amine est substituée par un ou deux radicaux, identiques ou différents, choisis parmi les radicaux alkyle(C₁-C₆), monohydroxyalkyle(C₁-C₆), polyhydroxyalkyle(C₂-C₆), alkyl(C₁-C₆)carbonyle, formyle, trifluoroalkyl(C₁-C₆)carbonyle, alkyl(C₁-C₆)carboxy, carbamyle, N-alkyl(C₁-C₆)carbamyle, N,N-dialkyl(C₁-C₆)carbamyle, thiocarbamyle, alkyl(C₁-C₆)sulfonyle, ou par un groupement Z ;
• R₄, R₅, R'₄, R'₅, identiques ou différents, représentent l'une des deux valences d'un bras de liaison B, un atome d'hydrogène ; un groupement Z ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical thiocarbamylalkyle en C₁-C₆ ; un radical trifluoroalkyle en C₁-C₆ ; un radical sulfoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical aminosulfonylalkyle en C₁-C₆ ; un radical N-Z-aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)carbonylalkyle en C₁-C₆ ; un radical aminoalkyle en C₁-C₆ ; un radical aminoalkyle(C₁-C₆) dont l'alkyle est substitué ou non substitué par un ou plusieurs radicaux hydroxy et dont l'amine est substituée par un ou deux radicaux, identiques ou différents, choisis parmi les radicaux alkyle, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle, ou par un groupement Z ;
• Z est choisi parmi les groupements cationiques insaturés de formules (Il) et (III) suivantes, et les groupements cationiques saturés de formule (IV) suivante : 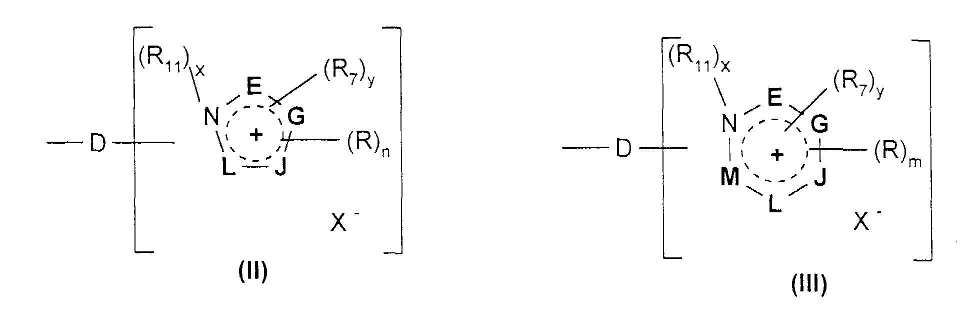 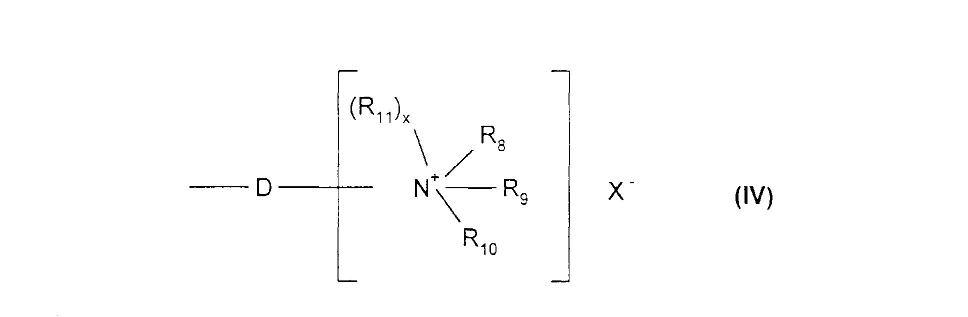
dans lesquelles :
• D est un bras de liaison qui représente une chaîne alkyle comportant de préférence de 1 à 14 atomes de carbone, linéaire ou ramifiée pouvant être interrompue par un ou plusieurs hétéroatomes tels que des atomes d'oxygène, de soufre ou d'azote, et pouvant être substituée par un ou plusieurs radicaux hydroxyle ou alcoxy en C₁-C₆, et pouvant porter une ou plusieurs fonctions cétone ;
• les sommets E, G, J, L et M, identiques ou différents, représentent un atome de carbone, d'oxygène, de soufre ou d'azote ;
• n est un nombre entier compris entre 0 et 4 inclusivement ;
• m est un nombre entier compris entre 0 et 5 inclusivement ;
• les radicaux R, identiques ou différents, représentent l'une des deux valences d'un bras de liaison B, un second groupement Z identique ou différent du premier groupement Z, un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle; un groupement NHR" ou NR"R"' dans lesquels R" et R"', identiques ou différents, représentent un radical alkyle en C₁-C₆, un radical monohydroxyalkyle en C₁-C₆ ou un radical polyhydroxyalkyle en C₂-C₆ ;
• R₇ représente l'une des deux valences d'un bras de liaison B, un radical alkyle en C₁-C₆, un radical monohydroxyalkyle en C₁-C₆, un radical polyhydroxyalkyle en C₂-C₆, un radical cyanoalkyle en C₁-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle, un second groupement Z identique ou différent du premier groupement Z ;
• R₈, R₉ et R₁₀, identiques ou différents, représentent l'une des deux valences d'un bras de liaison B, un radical alkyle en C₁-C₆, un radical monohydroxyalkyle en C₁-C₆, un radical polyhydroxyalkyle en C₂-C₆, un radical alcoxy(C₁-C₆)alkyle en C₁-C₆, un radical cyanoalkyle en C₁-C₆, un radical aryle, un radical benzyle, un radical amidoalkyle en C₁-C₆, un radical trialkyl(C₁-C₆)silanealkyle en C₁-C₆ ou un radical aminoalkyle en C₁-C₆ dont l'amine est protégée par un radical alkyl(C₁-C₆)carbonyle, carbamyle, ou alkyl(C₁-C₆)sulfonyle; deux des radicaux R₈, R₉ et R₁₀ peuvent également former ensemble, avec l'atome d'azote auquel ils sont rattachés, un cycle saturé à 5 ou 6 chaînons carboné pouvant contenir un ou plusieurs hétéroatomes tel que par exemple un cycle pyrrolidine, un cycle pipéridine, un cycle pipérazine ou un cycle morpholine, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C_{6,} un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical cétoalkyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ;
l'un des radicaux R₈, R₉ et R₁₀ peut également représenter un second groupement Z, identique ou différent du premier groupement Z ;
• R₁₁ représente l'une des deux valences d'un bras de liaison B, un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est protégée par un radical alkyl(C₁-C₆)carbonyle, carbamyle ou alkyl(C₁-C₆)sulfonyle ; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
• x et y sont des nombres entiers égaux à 0 ou 1 ; avec les conditions suivantes :
- dans les groupements cationiques insaturés de formule (II) :
- lorsque x = 0, le bras de liaison D est rattaché à l'atome d'azote,
- lorsque x = 1, le bras de liaison D est rattaché à l'un des sommets E, G, J ou L,
- y ne peut prendre la valeur 1 que :
1) lorsque les sommets E, G, J et L représentent simultanément un atome de carbone, et que le radical R₇ est porté par l'atome d'azote du cycle insaturé ; ou bien
2) lorsqu'au moins un des sommets E, G, J et L représente un atome d'azote sur lequel le radical R₇ est fixé ;
- dans les groupements cationiques insaturés de formule (III):
- lorsque x = 0, le bras de liaison D est rattaché à l'atome d'azote,
- lorsque x = 1, le bras de liaison D est rattaché à l'un des sommets E, G, J, L ou M,
- y ne peut prendre la valeur 1 que lorsqu'au moins un des sommets E, G, J, L et M représente un atome divalent, et que le radical R₇ est porté par l'atome d'azote du cycle insaturé ;
- dans les groupements cationiques de formule (IV) :
- lorsque x = 0, alors le bras de liaison D est rattaché à l'atome d'azote portant les radicaux R₈ à R₁₀,
- lorsque x = 1, alors deux des radicaux R₈ à R₁₀ forment conjointement avec l'atome d'azote auquel ils sont rattachés un cycle saturé à 5 ou 6 chaînons tel que défini précédemment; et le bras de liaison D est porté par un atome de carbone dudit cycle saturé ;
• X⁻ représente un anion monovalent ou divalent,
étant entendu que le nombre de groupement cationique Z est au moins égal à 1.

2. Utilisation selon la revendication 1, caractérisée par le fait que les cycles des groupements insaturés Z de formule (II) sont choisis parmi les cycles pyrrolique, imidazolique, pyrazolique, oxazolique, thiazolique et triazolique.

3. Utilisation selon la revendication 1, caractérisée par le fait que les cycles des groupements insaturés Z de formule (III) sont choisis parmi les cycles pyridinique, pyrimidinique, pyrazinique, oxazinique et triazinique.

4. Utilisation selon l'une quelconque des revendications précédentes, caractérisée par le fait que dans la formule (IV) deux des radicaux R₈, R₉ et R₁₀ forment un cycle pyrrolidinique, un cycle pipéridinique, un cycle pipérazinique ou un cycle morpholinique, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle.

5. Utilisation selon l'une quelconque des revendications précédentes, caractérisée par le fait que X⁻ est choisi parmi un atome d'halogène, un hydroxyde, un hydrogènesulfate, ou un alkyl(C₁-C₆)sulfate.

6. Utilisation selon l'une quelconque des revendications précédentes, caractérisée par le fait que les composés de formule (I) sont choisis parmi ceux de formules (I)₁ à (I)₂₃ suivantes :

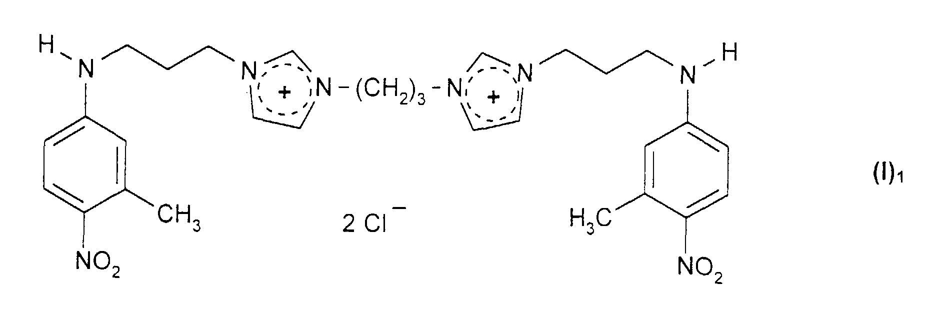

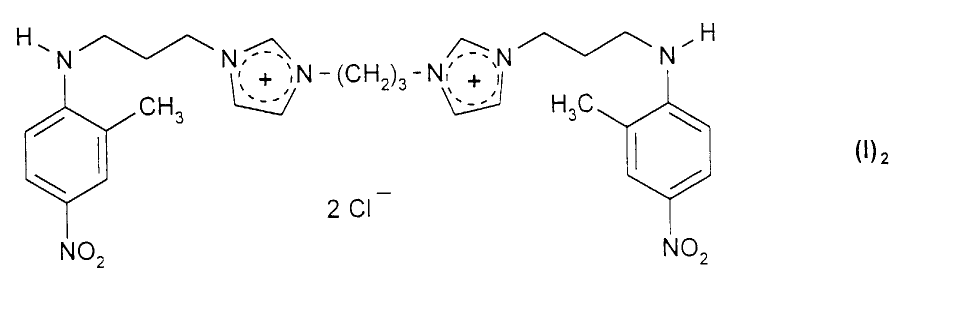

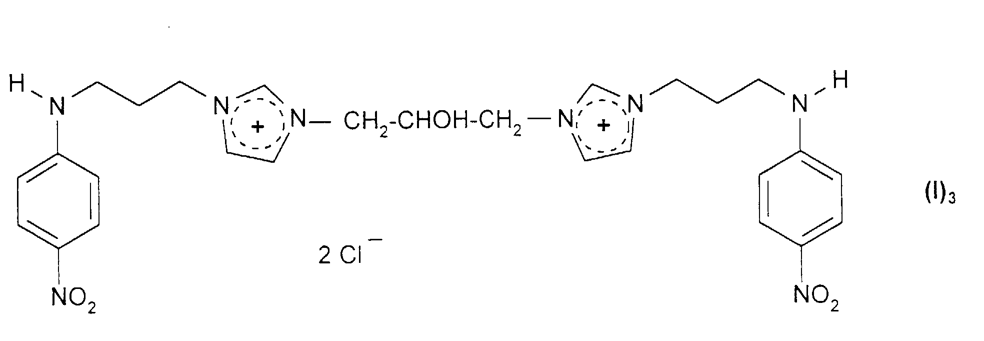

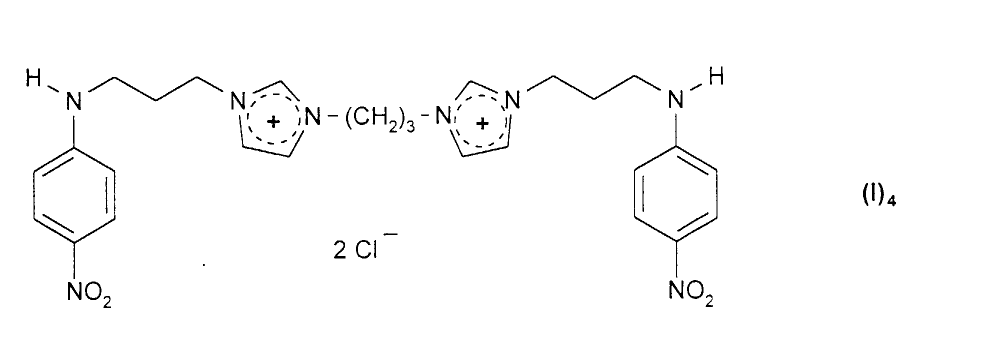

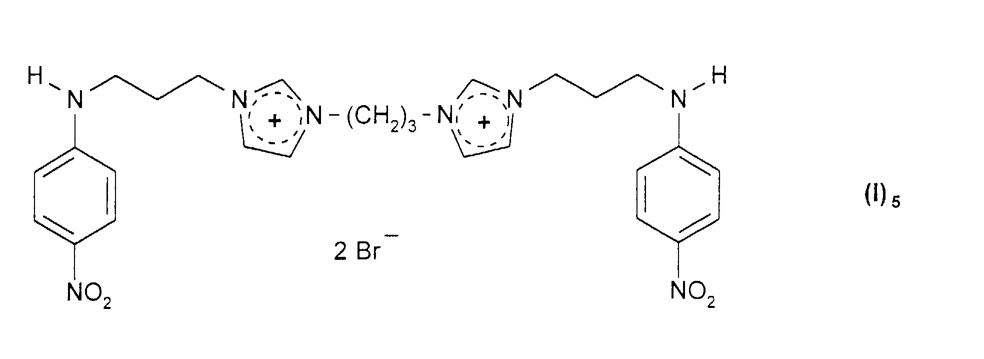

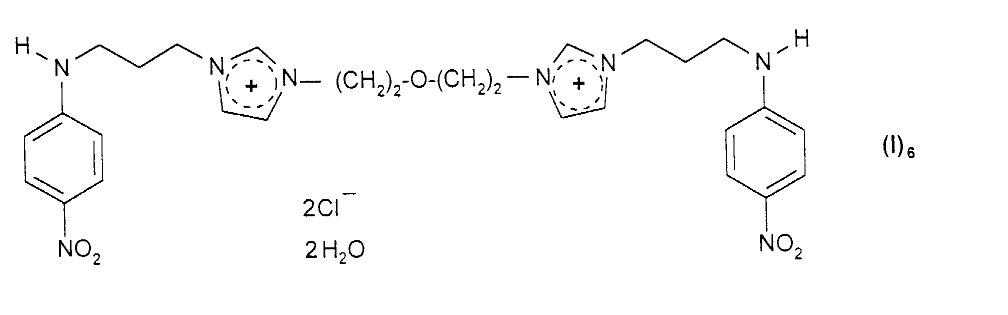

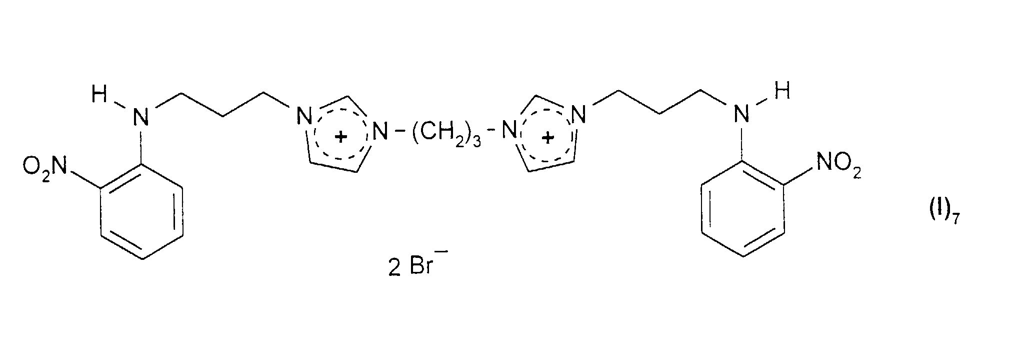

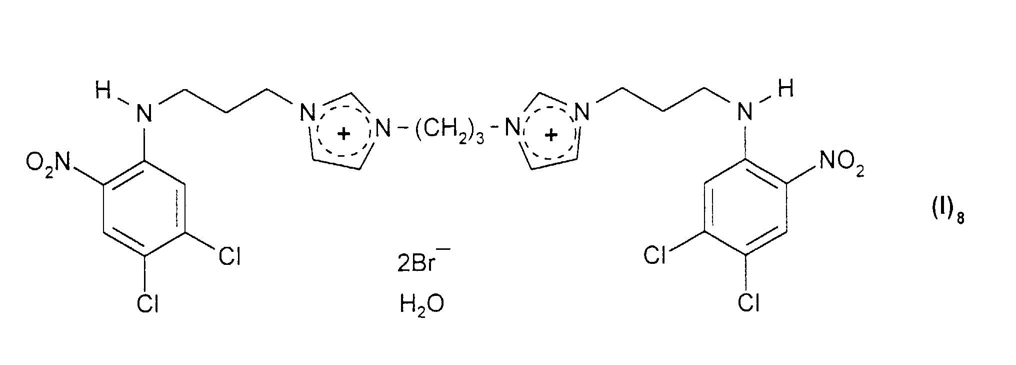

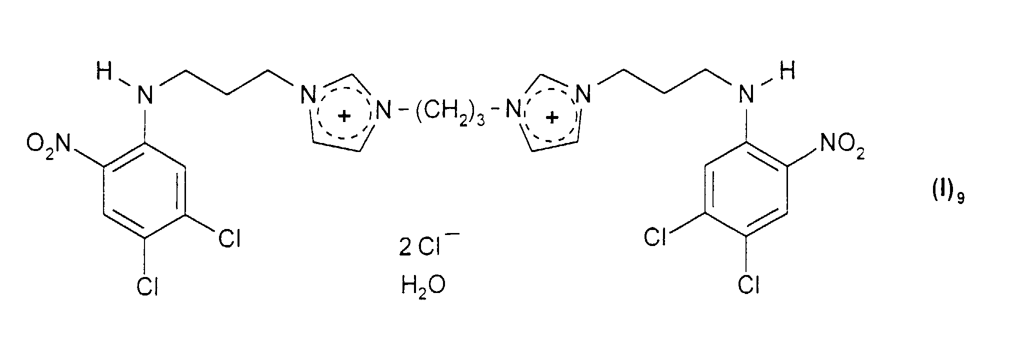

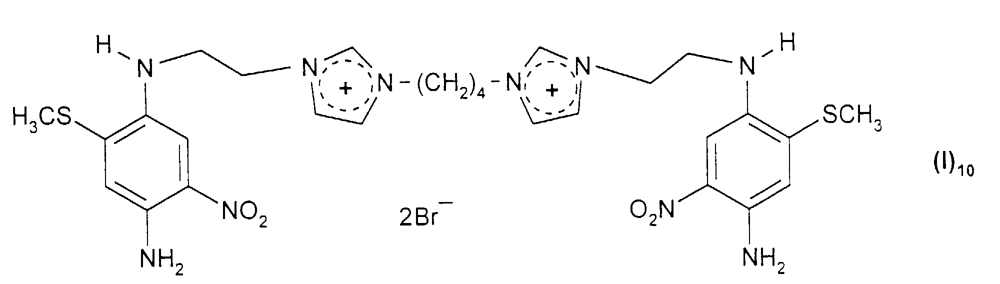

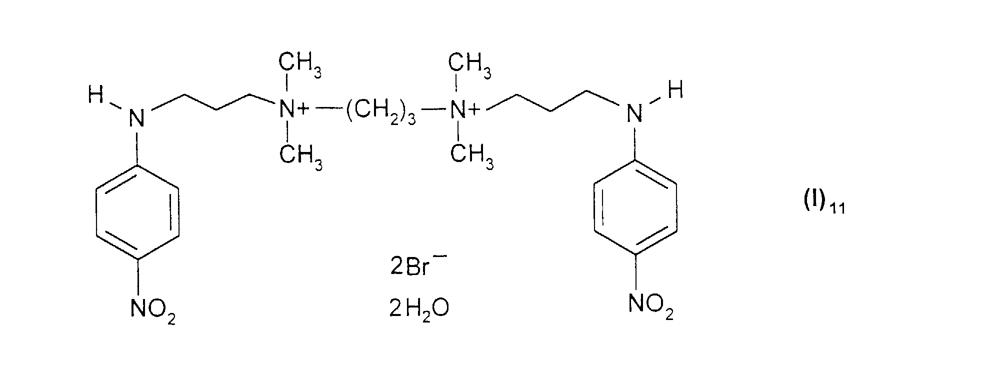

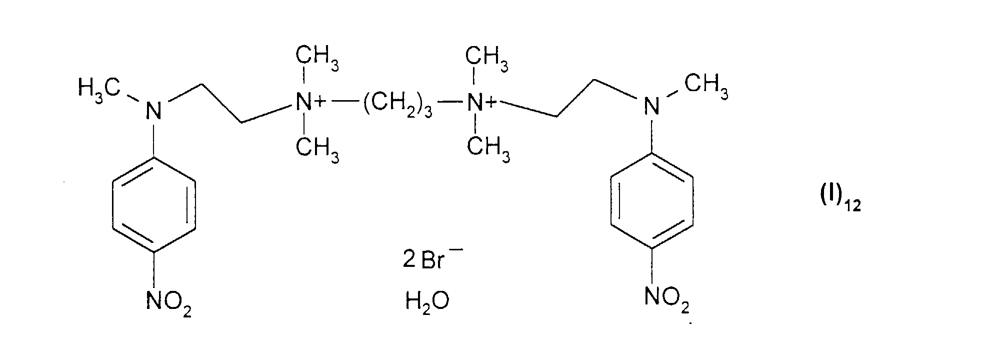

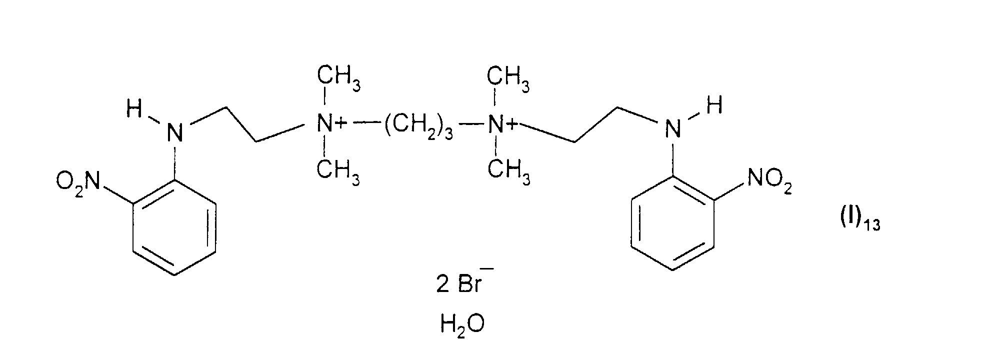

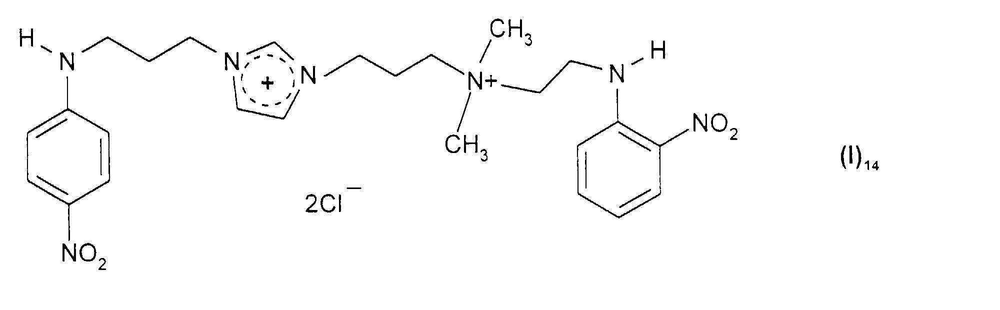

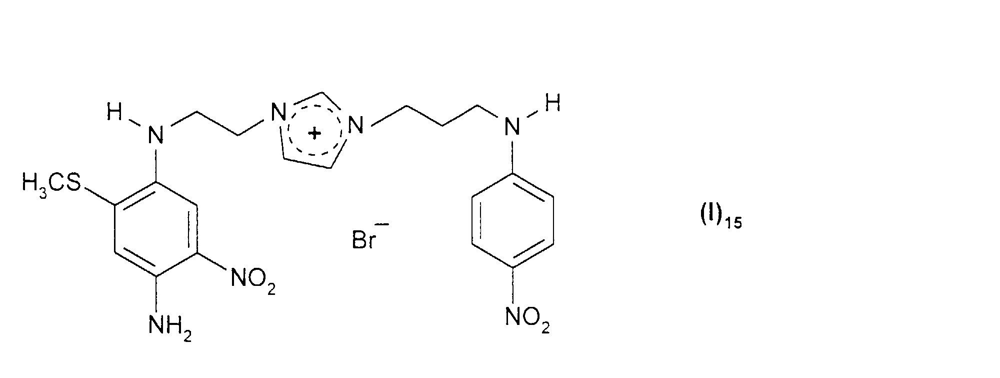

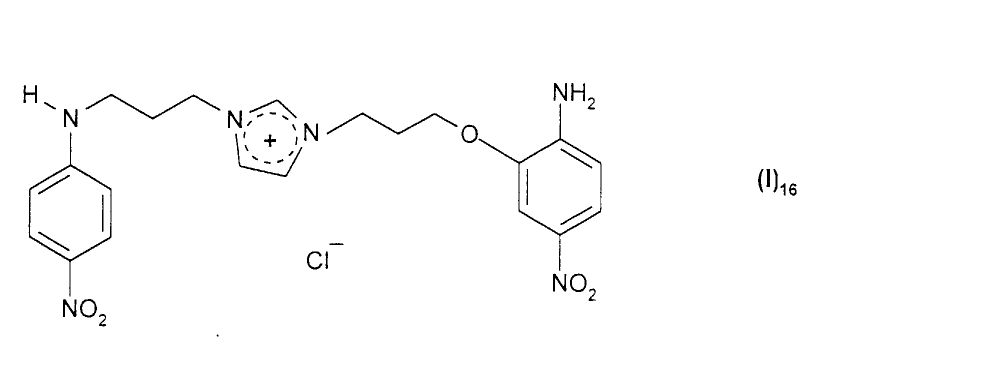

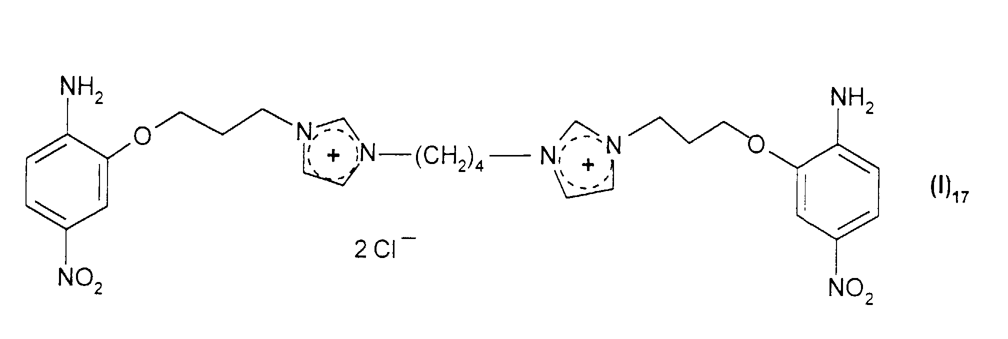

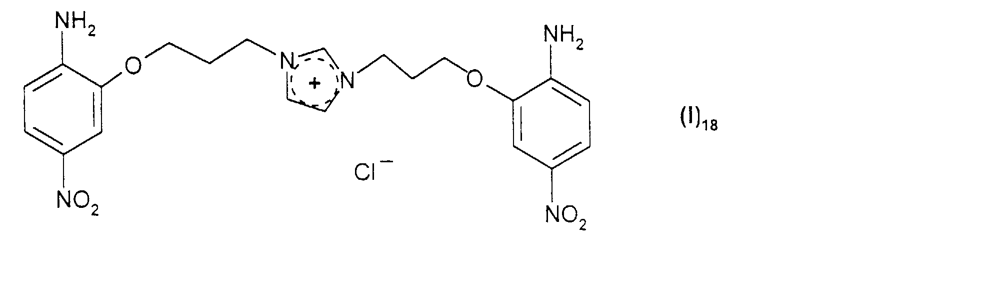

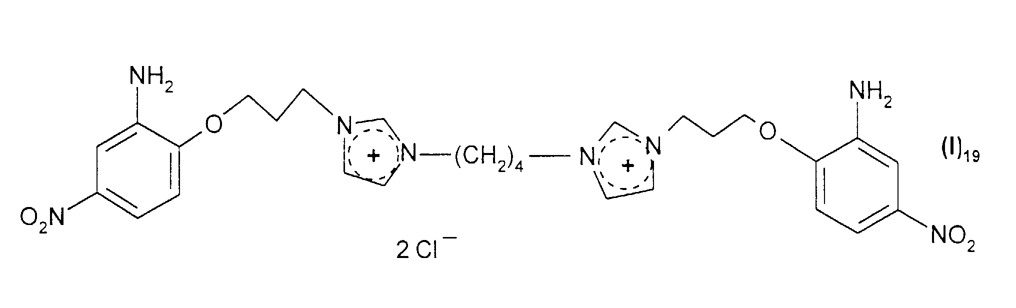

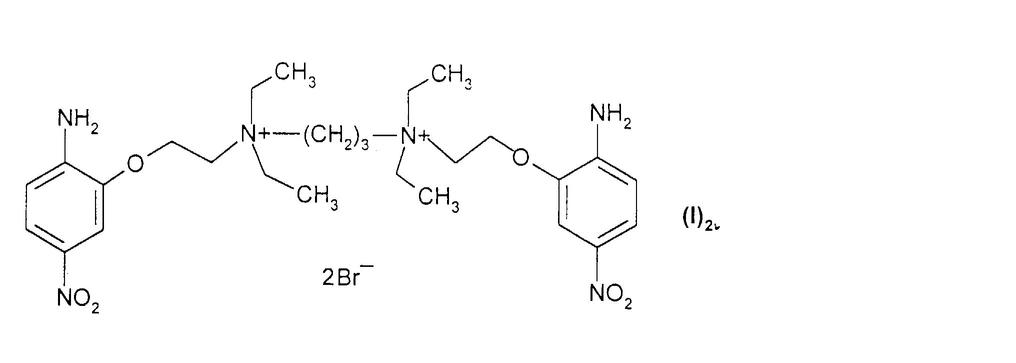

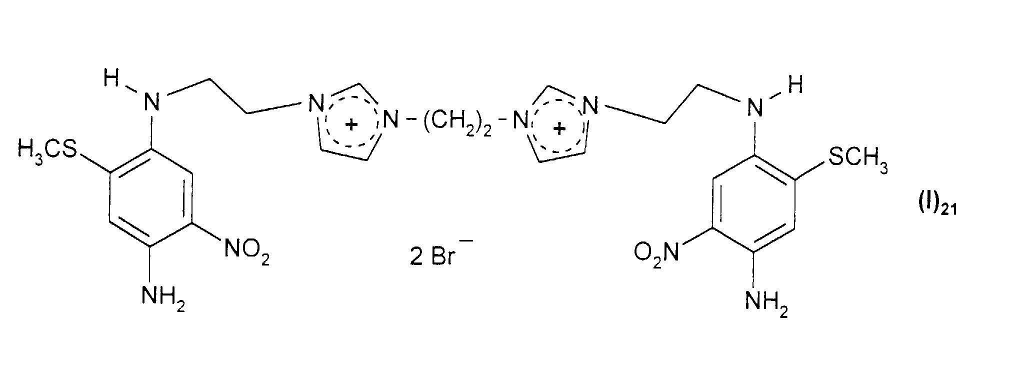

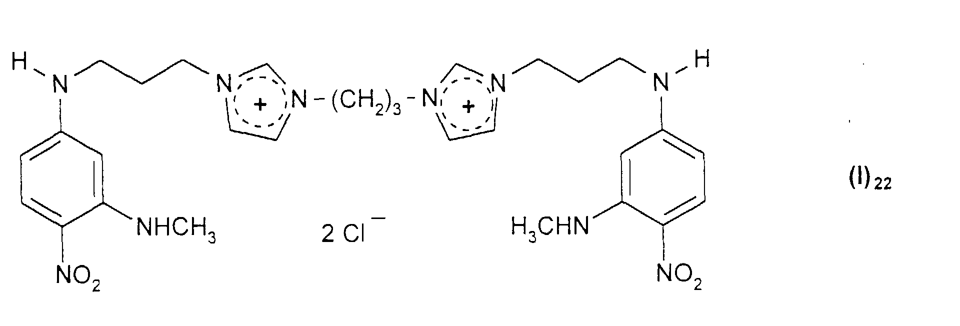

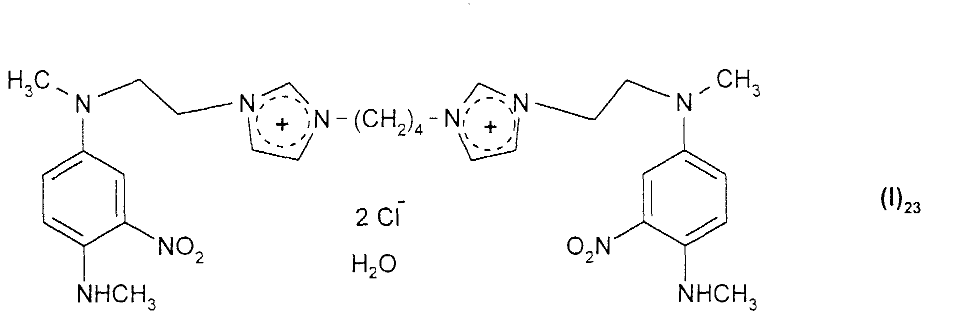

7. Utilisation selon l'une quelconque des revendications précédentes, caractérisée par le fait que les sels d'addition avec un acide des composés de formule (I) sont choisis parmi les chlorhydrates, les bromhydrates, les sulfates, les citrates, les succinates, les tartrates, les lactates et les acétates.

8. Composition tinctoriale pour matières kératiniques, caractérisée par le fait qu'elle comprend, dans un milieu approprié pour la teinture, une quantité efficace d'au moins un composé de formule (I) défini à l'une quelconque des revendications 1-6.

9. Composition de teinture directe pour fibres kératiniques humaines et notamment les cheveux, caractérisée par le fait qu'elle comprend, dans un milieu approprié pour la teinture, une quantité efficace d'au moins un composé de formule (I) défini à l'une quelconque des revendications 1-6.

10. Composition selon les revendications 8 ou 9, caractérisée par le fait qu'elle a un pH compris entre 3 et 12.

11. Composition selon l'une quelconque des revendications 8 à 10, caractérisée par le fait que les composés de formule (I) sont présents dans une concentration allant de 0,005 à 12 % en poids par rapport au poids total de la composition.

12. Composition selon la revendication 11, caractérisée par le fait que les composés de formule (I) sont présents dans une concentration allant de 0,05 à 6 % en poids par rapport au poids total de la composition.

13. Composition selon l'une quelconque des revendications 8 à 12, caractérisée par le fait que le milieu approprié pour la teinture est un milieu aqueux constitué par de l'eau et/ou des solvants organiques, dans des proportions comprises entre 1 et 40% en poids par rapport au poids total de la composition.

14. Procédé de teinture des fibres kératiniques et en particulier des fibres kératiniques humaines et notamment les cheveux, par coloration directe, caractérisé par le fait qu'on applique une composition tinctoriale telle que définie à l'une quelconque des revendications 8 à 13, sur les fibres kératiniques sèches ou humides, et qu'on sèche ces fibres sans rinçage intermédiaire.

15. Procédé de teinture des fibres kératiniques et en particulier des fibres kératiniques humaines et notamment les cheveux, par coloration directe, caractérisé par le fait qu'on applique une composition tinctoriale telle que définie à l'une quelconque des revendications 8 à 13 sur les fibres kératiniques sèches ou humides, et qu'après avoir éventuellement laissé agir la composition sur les fibres pendant un temps de pose allant de 3 à 60 minutes, on rince les fibres, on les lave éventuellement, on les rince à nouveau puis on les sèche.
